# EUROPEAN PATENT SPECIFICATION

(11) **EP 2 993 226 B1**
(45) Date of publication and mention of the grant of the patent: **16.12.2020**
(21) Application number: 15183719.2
(22) Date of filing: 03.06.2008
(51) Int. Cl.: C12N 5/0735, C12N 5/071, C12N 15/09, C12P 21/02

(54) **GROWTH FACTORS FOR PRODUCTION OF DEFINITIVE ENDODERM**
WACHSTUMSFAKTOREN ZUR HERSTELLUNG VON DEFINITIVEN ENDODERMEN
FACTEURS DE CROISSANCE POUR LA PRODUCTION D'UN ENDODERME DÉFINITIF

(43) Date of publication of application: 09.03.2016
(62) Divisional of application: 08756668.3
(73) Proprietor: VIACYTE, INC., San Diego, CA 92121 (US)
(72) Inventor: D'AMOUR, Kevin Allen, San Diego, CA 92106 (US)
(74) Representative: Vossius & Partner Patentanwälte Rechtsanwälte mbB

(56) References cited:
- WO-A2-2004/073633
- WO-A2-2005/045001
- WO-A2-2007/047509
- WO-A2-2011/011302
- D'AMOUR KEVIN A ET AL: "Efficient differentiation of human embryonic stem cells to definitive endoderm", NATURE BIOTECHNOLOGY, vol. 23, no. 12, December 2005 (2005-12), pages 1534-1541, XP002651213, ISSN: 1087-0156
- MCLEAN A B ET AL: "Activin A efficiently specifies definitive endoderm from human embryonic stem cells only when phosphatidylinositol 3-kinase signaling is suppressed", STEM CELLS 200701 US LNKD- DOI:10.1634/STEMCELLS.2006-0219, vol. 25, no. 1, January 2007 (2007-01), pages 29-38, XP002651214, ISSN: 1066-5099
- ALIREZA REZANIA ET AL: "Reversal of diabetes with insulin-producing cells derived in vitro from human pluripotent stem cells", NATURE BIOTECHNOLOGY, vol. 32, no. 11, 11 September 2014 (2014-09-11), pages 1121-1133, XP055200937, ISSN: 1087-0156, DOI: 10.1038/nbt.3033

### SEQUENCE LISTING

<110> VIACYTE, Inc.
<120> GROWTH FACTORS FOR PRODUCTION OF DEFINITIVE ENDODERM
<130> S3339 EP/1 S3
<150> EP 08 75 6668.3
   <151> 2008-06-03
<160> 36
<170> FastSEQ for Windows Version 4.0
<210> 1
   <211> 21
   <212> DNA
   <213> Artificial Sequence
<220>
   <221> Source
   <223> /note = "Description of Artificial Sequence: Primers"
<400> 1
   cttgtcaatg gccaacagag g 21
<210> 2
   <211> 23
   <212> DNA
   <213> Artifical Sequence
<220>
   <221> Source
   <223> /note = "Description of Artificial Sequence: Primers"
<400> 2
   gcccatctaa atgaggagtt ggt 23
<210> 3
   <211> 20
   <212> DNA
   <213> Artificial Sequence
<220>
   <221> Source
   <223> /note = "Description of Artificial Sequence: Primers"
<400> 3
   tgtgcacagg agccaagagt 20
<210> 4
   <211> 21
   <212> DNA
   <213> Artificial Sequence
<220>
   <221> Source
   <223> /note = "Description of Artificial Sequence: Primers"
<400> 4
   attttcttgc tgccagtctg g 21
<210> 5
   <211> 21
   <212> DNA
   <213> Artifical Sequence
<220>
   <221> Source
   <223> /note = "Description of Artificial Sequence: Primers"
<400> 5
   aagaggccat caagcagatc a 21
<210> 6
   <211> 17
   <212> DNA
   <213> Artificial Sequence
<220>
   <221> Source
   <223> /note = "Description of Artificial Sequence: Primers"
<400> 6
   caggaggcgc atccaca 17
<210> 7
   <211> 20
   <212> DNA
   <213> Artificial Sequence
<220>
   <221> Source
   <223> /note = "Description of Artificial Sequence: Primers"
<400> 7
   ctggcctgta cccctcatca 20
<210> 8
   <211> 21
   <212> DNA
   <213> Artificial Sequence
<220>
   <221> Source
   <223> /note = "Description of Artificial Sequence: Primers"
<400> 8
   cttcccgtct ttgtccaaca a 21
<210> 9
   <211> 21
   <212> DNA
   <213> Artificial Sequence
<220>
   <221> Source
   <223> /note = "Description of Artificial Sequence: Primers"
<400> 9
   aagtctacca aagctcacgc g 21
<210> 10
   <211> 15
   <212> DNA
   <213> Artificial Sequence
<220>
   <221> Source
   <223> /note = "Description of Artificial Sequence: Primers"
<400> 10
   gtaggcgccg cctgc 15
<210> 11
   <211> 24
   <212> DNA
   <213> Artificial Sequence
<220>
   <221> Source
   <223> /note = "Description of Artificial Sequence: Primers"
<400> 11
   gctcatcgct ctctattctt ttgc 24
<210> 12
   <211> 21
   <212> DNA
   <213> Artificial Sequence
<220>
   <221> Source
   <223> /note = "Description of Artificial Sequence: Primers"
<400> 12
   ggttgaggcg tcatcctttc t 21
<210> 13
   <211> 18
   <212> DNA
   <213> Artificial Sequence
<220>
   <221> Source
   <223> /note = "Description of Artificial Sequence: Primers"
<400> 13
   gggagcggtg aagatgga 18
<210> 14
   <211> 22
   <212> DNA
   <213> Artificial Sequence
<220>
   <221> Source
   <223> /note = "Description of Artificial Sequence: Primers"
<400> 14
   tcatgttgct cacggaggag ta 22
<210> 15
   <211> 24
   <212> DNA
   <213> Artificial Sequence
<220>
   <221> Source
   <223> /note = "Description of Artificial Sequence: Primers"
<400> 15
   aagcatttac tttgtggctg gatt 24
<210> 16
   <211> 25
   <212> DNA
   <213> Artificial Sequence
<220>
   <221> Source
   <223> /note = "Description of Artificial Sequence: Primers"
<400> 16
   tgatctggat ttctcctctg tgtct 25
<210> 17
   <211> 18
   <212> DNA
   <213> Artificial Sequence
<220>
   <221> Source
   <223> /note = "Description of Artificial Sequence: Primers"
<400> 17
   cgctccgctt agcagcat 18
<210> 18
   <211> 22
   <212> DNA
   <213> Artificial Sequence
<220>
   <221> Source
   <223> /note = "Description of Artificial Sequence: Primers"
<400> 18
   gtgttgcctc tatccttccc at 22
<210> 19
   <211> 20
   <212> DNA
   <213> Artificial Sequence
<220>
   <221> Source
   <223> /note = "Description of Artificial Sequence: Primers"
<400> 19
   gaagaaggaa gccgtccaga 20
<210> 20
   <211> 20
   <212> DNA
   <213> Artificial Sequence
<220>
   <221> Source
   <223> /note = "Description of Artificial Sequence: Primers"
<400> 20
   gaccttcgag tgctgatccg 20
<210> 21
   <211> 18
   <212> DNA
   <213> Artificial Sequence
<220>
   <221> Source
   <223> /note = "Description of Artificial Sequence: Primers"
<400> 21
   ggcgcagcag aatccaga 18
<210> 22
   <211> 18
   <212> DNA
   <213> Artificial Sequence
<220>
   <221> Source
   <223> /note = "Description of Artificial Sequence: Primers"
<400> 22
   ccacgacttg cccagcat 18
<210> 23
   <211> 16
   <212> DNA
   <213> Artificial Sequence
<220>
   <221> Source
   <223> /note = "Description of Artificial Sequence: Primers"
<400> 23
   caccgcgggc atgatc 16
<210> 24
   <211> 19
   <212> DNA
   <213> Artificial Sequence
<220>
   <221> Source
   <223> /note = "Description of Artificial Sequence: Primers"
<400> 24
   acttccccag gaggttcga 19
<210> 25
   <211> 20
   <212> DNA
   <213> Artificial Sequence
<220>
   <221> Source
   <223> /note = "Description of Artificial Sequence: Primers"
<400> 25
   ggccttcagt actccctgca 20
<210> 26
   <211> 21
   <212> DNA
   <213> Artificial Sequence
<220>
   <221> Source
   <223> /note = "Description of Artificial Sequence: Primers"
<400> 26
   gggacttgga gcttgagtcc t 21
<210> 27
   <211> 21
   <212> DNA
   <213> Artificial Sequence
<220>
   <221> Source
   <223> /note = "Description of Artificial Sequence: Primers"
<400> 27
   gaaggtcatc atctgccatc g 21
<210> 28
   <211> 19
   <212> DNA
   <213> Artificial Sequence
<220>
   <221> Source
   <223> /note = "Description of Artificial Sequence: Primers"
<400> 28
   ggccataatc agggtcgct 19
<210> 29
   <211> 20
   <212> DNA
   <213> Artificial Sequence
<220>
   <221> Source
   <223> /note = "Description of Artificial Sequence: Primers"
<400> 29
   ccccagactc cgtcagtttc 20
<210> 30
   <211> 19
   <212> DNA
   <213> Artificial Sequence
<220>
   <221> Source
   <223> /note = "Description of Artificial Sequence: Primers"
<400> 30
   tccgtctggt tgggttcag 19
<210> 31
   <211> 23
   <212> DNA
   <213> Artificial Sequence
<220>
   <221> Source
   <223> /note = "Description of Artificial Sequence: Primers"
<400> 31
   ccagaaagga tgcctcataa agg 23
<210> 32
   <211> 18
   <212> DNA
   <213> Artificial Sequence
<220>
   <221> Source
   <223> /note = "Description of Artificial Sequence: Primers"
<400> 32
   tctgcgcgcc cctagtta 18
<210> 33
   <211> 20
   <212> DNA
   <213> Artificial Sequence
<220>
   <221> Source
   <223> /note = "Description of Artificial Sequence: Primers"
<400> 33
   gggctctctg agaggcaggt 20
<210> 34
   <211> 19
   <212> DNA
   <213> Artificial Sequence
<220>
   <221> Source
   <223> /note = "Description of Artificial Sequence: Primers"
<400> 34
   cctttgctct gcggttctg 19
<210> 35
   <211> 19
   <212> DNA
   <213> Artificial Sequence
<220>
   <221> Source
   <223> /note = "Description of Artificial Sequence: Primers"
<400> 35
   acgccgagct cagcaagat 19
<210> 36
   <211> 20
   <212> DNA
   <213> Artificial Sequence
<220>
   <221> Source
   <223> /note = "Description of Artificial Sequence: Primers"
<400> 36
   tccacgtacg gcctcttctg 20

## Description

### REFERENCE TO SEQUENCE LISTING

The present application is being filed along with a Sequence Listing in electronic format. The Sequence listing is provided as a text file entitled CYTHERA064VPC.TXT, created May 28, 2008, which is 6.06 KB in size.

### FIELD OF THE INVENTION

The present invention relates to the fields of medicine and cell biology. In particular, the present invention relates to compositions and methods for generating endoderm lineage type cells from human pluripotent cells as well as cell compositions produced by these methods.

### BACKGROUND

Human embryonic stem (hES) cells are uniquely suited for cell therapy applications because they are pluripotent and self-renewable. Owing to the large variety of cell types that can arise in differentiating pluripotent hES cell cultures, success in achieving efficient, directed differentiation is useful for therapeutic application of hESCs. Efficient directed differentiation of hES cells to various intermediate cell types, as well as pancreatic islet beta-cell lineages, using various growth and signaling factors is necessary.

### SUMMARY OF THE INVENTION

Some embodiments of the present invention relate to methods for generating various hES-derived cell populations, including definitive endoderm, a multipotent cell that can differentiate into cells of the gut tube or organs derived from the gut tube, e.g., the pancreas, using various TGF-beta super family protein members alone or in combination with Wnt3a protein and/or small molecule GSK3-beta inhibitors.

The present invention relates to a method for generating pancreatic endoderm cells, said method comprising:
(a) providing an *in vitro* cell population comprising pluripotent human cells with a factor selected from the group consisting of GDF8, GDF11 and a combination of GDF8 and GDF11 in an amount sufficient to induce pluripotent human cells of said cell population to differentiate into definitive endoderm cells, wherein said amount is at least 100 ng/ml GDF8 or at least 100 ng/ml GDF11 or at least 100 ng/ml of a combination of GDF8 and GDF11, and is sufficient to produce an *in vitro* cell population comprising at least 15% human definitive endoderm cells,
(b) further culturing the pluripotent-derived cells of step (a) to induce differentiation of the cells to pancreatic endoderm cells.

24. An *in vitro* human cell culture comprising:
an *in vitro* cell population comprising human pluripotent cells and a factor selected from the group consisting of GDF8, GDF11 and a combination of GDF8 and GDF11; further comprising human definitive endoderm cells wherein at least 10% of the human cells in said cell culture are human definitive endoderm cells and wherein, said factor is at least 100 ng/ml GDF8 or at least 100 ng/ml GDF11 or at least 100 ng/ml of a combination of GDF8 and GDF11; and pancreatic endoderm cells.

It will be appreciated that the methods and compositions described above relate to cells cultured *in vitro.* However, the above-described *in vitro* differentiated cell compositions may be used for *in vivo* applications.

Processes and compositions related to but distinct from the present invention can be found in United States Provisional Patent Application No. 60/532,004, entitled DEFINITIVE ENDODERM, filed December 23, 2003; U.S. Provisional Patent Application Number 60/566,293, entitled PDX1 EXPRESSING ENDODERM, filed April 27, 2004; U.S. Provisional Patent Application Number 60/586,566, entitled CHEMOKINE CELL SURFACE RECEPTOR FOR THE ISOLATION OF DEFINITIVE ENDODERM, filed July 9, 2004; U.S. Provisional Patent Application Number 60/587,942, entitled CHEMOKINE CELL SURFACE RECEPTOR FOR THE ISOLATION OF DEFINITIVE ENDODERM, filed July 14, 2004; U.S. Patent Application Number 11/021,618, entitled DEFINITIVE ENDODERM, filed December 23, 2004 and U.S. Patent Application Number 11/115,868, entitled PDX1 EXPRESSING ENDODERM, filed April 26, 2005; U.S. Patent Application Number 11/165,305, entitled METHODS FOR IDENTIFYING FACTORS FOR DIFFERENTIATING DEFINITIVE ENDODERM, filed June 23, 2005; U.S. Provisional Patent Application Number 60/730,917, entitled PDX1-EXPRESSING DORSAL AND VENTRAL FOREGUT ENDODERM, filed October 27, 2005; U.S. Provisional Patent Application Number 60/736,598, entitled MARKERS OF DEFINITIVE ENDODERM, filed November 14, 2005; U.S. Provisional Patent Application Number 60/778,649, entitled INSULIN-PRODUCING CELLS AND METHOD OF PRODUCTION, filed March 2, 2006; U.S. Provisional Patent Application Number 60/833,633, entitled INSULIN-PRODUCING CELLS AND METHOD OF PRODUCTION, filed July 26, 2006; U.S. Provisional Patent Application Number 60/852,878, entitled ENRICHMENT OF ENDOCRINE PRECURSOR CELLS, IMMATURE PANCREATIC ISLET CELLS AND MATURE PANCREATIC ISLET CELLS USING NCAM, filed October 18, 2006; U.S. Patent Application Number 11/588,693, entitled PDX1-EXPRESSING DORSAL AND VENTRAL FOREGUT ENDODERM, filed October 27, 2006; U.S. Patent Application Number 11/681,687, entitled ENDOCRINE PRECURSOR CELLS, PANCREATIC HORMONE-EXPRESSING CELLS AND METHODS OF PRODUCTION, filed March 2, 2007; U.S. Patent Application Number 11/773,944, entitled METHODS OF PRODUCING PANCREATIC HORMONES, filed July 5, 2007; U.S. Patent Application Number 60/972,174, entitled METHODS OF TREATMENT FOR DIABETES, filed September 13, 2007; U.S. Patent Application Number 11/860,494, entitled METHODS FOR INCREASING DEFINITIVE ENDODERM PRODUCTION, filed September 24, 2007; U.S. Patent Application Number 60/977,349, entitled CELL SURFACE MARKERS OF HUMAN EMBRYONIC STEM CELLS AND CANCER STEM CELLS, filed October 3, 2007; and U.S. Patent Application Number 12/099,759, entitled METHODS OF PRODUCING PANCREATIC HORMONES, filed April 8, 2008; and U.S. Patent Application Number 12/107,020, entitled METHODS FOR PURIFYING ENDODERM AND PANCREATIC ENDODERM CELLS DERIVED FORM HUMAN EMBRYONIC STEM CELLS, filed April 21, 2008.

### BRIEF DESCRIPTION OF THE DRAWINGS

Figure 1 is a depiction of the phylogenetic relationship of TGF-beta family ligands. Figure 1 is adapted from Mazerbourg et al. (2005) Biol Chem 280(37): 32122-32132.
Figures 2A-E are bar charts showing the expression of brachyury, ISL1, SOX17, FOXA2, and SOX7 as determined by real-time PCR.
Figures 3A-C are bar charts showing the expression of PDX1, PTF1A, and NKX2-2 as determined by real-time PCR.
Figures 4A-C are bar charts showing the expression of SOX17, CER1, and CDX2 as determined by real-time PCR.
Figures 5A-D are bar charts showing the expression of PDX1, PTF1A, NKX6-1 and NKX2-2 as determined by real-time PCR.
Figures 6A-C are bar charts showing the expression of SOX17, FOXA2, and brachyury as determined by real-time PCR.
Figures 7A-D are bar charts showing the expression of PDX1, PTF1A, NKX6-1 and NKX2-2 as determined by real-time PCR.
Figures 8A-D are flow cytometry dot plots showing the relative number of CXCR4-positive cells as a proportion of total intact cell number.
Figures 9A-C are bar charts showing the expression of brachyury, SOX17, and CER1 as determined by real-time PCR.

### DETAILED DESCRIPTION

Described herein are compositions and methods for modulating the proliferation, survival and/or differentiation of pluripotent stem cells. Additionally, various ligands, proteins, polypeptides or functional fragments thereof, which have the ability to modulate proliferation, survival and/or differentiation of pluripotent stem cells; in particular, differentiation of pluripotent stem cells to definitive endoderm cells are described. In some embodiments, the subject ligands have the ability to modulate intracellular signal transduction pathways mediated by receptors for members of the Transforming Growth Factor-beta (TGF-beta) superfamily or Wnt family of molecules.

General methods for production of endoderm lineage cells derived from hES cells are described in related U.S. applications as indicated above, and D'Amour et al. 2005 Nat Biotechnol. 23:1534-41 and D'Amour et al. 2006 Nat Biotechnol. 24(11):1392-401.

For convenience, certain terms employed in the specification, examples, and appended claims are collected here. Unless defined otherwise, all technical and scientific terms used herein have the same meaning as commonly understood by one of ordinary skill in the art to which this invention belongs.

### Definitions

The practice of some embodiments presented herein will employ, unless otherwise indicated, conventional techniques of chemistry, molecular biology, recombinant DNA and immunology, which are within the capabilities of a person of ordinary skill in the art. Such techniques are explained in the literature. See, for example, J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements; Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons; J. M. Polak and James O'D. McGee, 1990, In Situ Hybridization: Principles and Practice; Oxford University Press; M. J. Gait (Editor), 1984, Oligonucleotide Synthesis: A Practical Approach, Irl Press; D. M. J. Lilley and J. E. Dahlberg, 1992, Methods of Enzymology: DNA Structure Part A: Synthesis and Physical Analysis of DNA Methods in Enzymology, Academic Press; and E. M. Shevach and W. Strober, 1992 and periodic supplements, Current Protocols in Immunology, John Wiley & Sons, New York, N.Y.

It will be appreciated that the numerical ranges expressed herein include the endpoints set forth and describe all integers between the endpoints of the stated numerical range.

hESC as disclosed herein can be derived from a "pre-implantation embryo". "Pre-implantation embryo" refers to an embryo between the stages of fertilization and implantation. Thus, a pre-implantation emoryo typically has not progressed beyond the blastocyst stage. Implantation of human embryos usually takes place 7-8 days after fertilization. However, implantation can take place about 2, about 3, about 4, about 5, about 6, about 7, about 8, about 9, about 10, about 11, about 12, about 13, about 14 or greater than about 14 days after fertilization.

As used herein, "multipotent" or "multipotent cell" refers to a cell type that can give rise to a limited number of other particular cell types. Multipotent cells are committed to one or more embryonic cell fates, and thus, in contrast to pluripotent cells, cannot give rise to each of the three embryonic cell lineages as well as extraembryonic cells.

In some embodiments, "pluripotent cells" are used as the starting material for pancreatic islet hormone-expressing cell differentiation. By "pluripotent" is meant that the cell can give rise to each of the three embryonic cell lineages as well as extraembryonic cells. Pluripotent cells, however, may not be capable of producing an entire organism.

In certain embodiments, the pluripotent cells used as starting material are stem cells, including human embryonic stem cells. As used herein, "embryonic" refers to a range of developmental stages of an organism beginning with a single zygote and ending with a multicellular structure that no longer comprises pluripotent or totipotent cells other than developed gametic cells.

"human embryonic stem cells" or "hES cells" or "hESCs" or "stem cells" or "pluripotent stem cells" are cells that can be obtained from an animal (e.g., a primate, such as a human) embryo. These terms and phrases can be equivalent to the phrase, "differentiable cell." A "differentiable cell" is used to describe a cell or population of cells that can differentiate into at least partially mature cells, or that can participate in the differentiation of cells, e.g., fuse with other cells, that can differentiate into at least partially mature cells.

As used herein, the phrase, "differentiable cell" or "differentiated cell" or "hES-derived cell" can be pluripotent, multipotent, oligopotent or even unipotent, as defined in detail below. In the disclosure, the differentiable cells are pluripotent differentiable cells. In the disclosure the pluripotent differentiable cells are selected from the group consisting of embryonic stem cells, ICM/epiblast cells, primitive ectoderm cells, primordial germ cells, and teratocarcinoma cells. In the disclosure, the differentiable cells are mammalian embryonic stem cells. The disclosure contemplates using differentiable cells from any animal capable of generating differentiable cells. The animals from which the differentiable cells are harvested can be vertebrate or invertebrate, mammalian or non-mammalian, human or non-human. Examples of animal sources include, but are not limited to, primates, rodents, canines, felines, equines, bovines and porcines. In the disclosure, the differentiable cells are human embryonic stem cells. Differentiable cells can also be harvested from embryos, or any primordial germ layer therein, from placental or chorion tissue, or from more mature tissue such as adult stem cells including, but not limited to adipose, bone marrow, nervous tissue, mammary tissue, liver tissue, pancreas, epithelial, respiratory, gonadal and muscle tissue. In the disclosure, the differentiable cells are embryonic stem cells. In other specific embodiments, the differentiable cells are adult stem cells. In still other specific embodiments, the stem cells are placental- or chorionic-derived stem cells.

As used herein, the term "differentiate" refers to the production of a cell type that is more differentiated than the cell type from which it is derived. The term therefore encompasses cell types that are partially and terminally differentiated. Similarly, "produced from hES cells," "derived from hES cells," "differentiated from hES cells," "hES derived cell" and equivalent expressions refer to the production of a differentiated cell type from hES cells in vitro and in vivo.

The differentiation culture conditions and hES-derived cell types described herein are substantially similar to that described in D'Amour et al. 2006, supra. D'Amour et al. describe a 5 step differentiation protocol: stage 1 (results in mostly definitive endoderm production), stage 2 (results in mostly PDX1-negative foregut endoderm production), stage 3 (results in mostly PDX1-positive foregut endoderm production), stage 4 (results in mostly pancreatic endoderm or pancreatic endocrine progenitor production) and stage 5 (results in mostly hormone expressing endocrine cell production).

As used herein, "definitive endoderm (DE)" refers to a multipotent endoderm lineage cell that can differentiate into cells of the gut tube or organs derived from the gut tube. In accordance with certain embodiments, the definitive endoderm cells are mammalian cells, and in a preferred embodiment, the definitive endoderm cells are human cells. In some embodiments of the present invention, definitive endoderm cells express or fail to significantly express certain markers. In some embodiments, one or more markers selected from SOX17, CXCR4, MIXL1, GATA4, HNF3beta, GSC, FGF17, VWF, CALCR, FOXQ1, CMKOR1 and CRIP1 are expressed in definitive endoderm cells. In other embodiments, one or more markers selected from OCT4, alpha-fetoprotein (AFP), Thrombomodulin (TM), SPARC, SOX7 and HNF4alpha are not significantly expressed in definitive endoderm cells. Definitive endoderm cell populations and methods of production thereof are also described in U.S. Application Number 11/021,618, entitled DEFINITIVE ENDODERM, filed December 23, 2004.

Still other embodiments relate to cell cultures termed "PDX1-negative foregut endoderm cells" or "foregut endoderm cells" or equivalents thereof. In some embodiments, the foregut endoderm cells express SOX17, HNF1beta and FOXA1 markers but do not substantially express PDX1, AFP, SOX7, SOX1. PDX1-negative foregut endoderm cell populations and methods of production thereof are also described in U.S. Application Number 11/588,693, entitled PDX1-expressing dorsal and ventral foregut endoderm, filed October 27, 2006.

Other embodiments of the present invention relate to cell cultures of "PDX1-positive, dorsally-biased, foregut endoderm cells" (dorsal PDX1-positive foregut endoderm cells) or just "PDX1-positive endoderm". In some embodiments, the PDX1-positive endoderm cells express one or more markers selected from Table 3 and/or one or more markers selected from Table 4 of related U.S. Application 11/588,693 entitled PDX1 EXPRESSING DOSAL AND VENTRAL FOREGUT ENDODERM, filed October 27, 2006, and also U.S. Application Number 11/115,868, entitled PDX1-expressing endoderm, filed April 26, 2005.

Additional embodiments, relate to cell cultures of PDX1-positive, ventrally-biased, foregut endoderm cells (ventral PDX1-positive foregut endoderm cells). In some embodiments, the PDX1-positive, ventrally-biased, foregut endoderm cells express one or more markers selected from Table 3 but do not substantially express a marker selected from Table 4 of related U.S. Application 11/588,693 above, as compared to the expression of the same marker in PDX1-positive, dorsally-biased, foregut endoderm cells.

The PDX1-positive foregut endoderm cells, such as those produced according to the methods described herein, are progenitors which can be used to produce fully differentiated pancreatic hormone secreting or endocrine cells, e.g., insulin-producing β-cells. In some embodiments of the present invention, PDX1-positive foregut endoderm cells are produced by differentiating definitive endoderm cells that do not substantially express PDX1 (PDX1-negative definitive endoderm cells; also referred to herein as definitive endoderm) so as to form PDX1-positive foregut endoderm cells.

As used herein, "pancreatic endoderm," pancreatic epithelial," "pancreatic epithelium (PE)," "pancreatic endocrine progenitor," "immature endocrine cell", or equivalents thereof are cells that exhibit at least one characteristic of the phenotype, such as morphology or protein expression, of a mature cell from the same organ or tissue, e.g., a pancreatic endocrine progenitor cell that expresses at least one marker phenotype of a mature pancreatic endocrine cell. These cell types are derived from a human pluripotent cell in vitro, which is at least capable of maturing into a pancreatic hormone endocrine secreting cell in vivo or in vitro. Pancreatic endoderm derived from hES cells in this manner are distinguished from other endodermal lineage cell types based on differential or high levels of expression of markers selected from PDX1, NKX6.1, PTF1A, CPA1, cMYC, NGN3, PAX4, ARX and NKX2.2 markers, but do not substantially express genes which are hallmark of pancreatic endocrine cells, for example, CHGA, INS, GCG, GHRL, SST, MAFA, PCSK1 and GLUT1. Additionally, some "endocrine progenitor cells" expressing NGN3 can differentiate into other non-pancreatic structures (e.g., duodenum). In one embodiment, the NGN3 expressing endocrine progenitor described herein differentiates into mature pancreatic lineage cells, e.g., pancreatic endocrine cells. Pancreatic endoderm or endocrine progenitor cell populations and methods thereof are also described in U.S. Application Number 11/773,944, entitled Methods of producing pancreatic hormones, filed July 5, 2007, and U.S. Application Number 12/107,020, entitled METHODS FOR PURIFYING ENDODERM AND PANCREATIC ENDODERM CELLS DERIVED FORM HUMAN EMBRYONIC STEM CELLS, filed April 21,2008.

As used herein, "pancreatic islet hormone-expressing cell," "pancreatic endocrine cell," or equivalents thereof refer to a cell, which has been derived from a pluripotent cell in vitro, which can be polyhormonal or singly-hormonal. The endocrine cells can therefore express one or more pancreatic hormones, which has at least some of the functions of a human pancreatic islet cell. Pancreatic islet hormone-expressing cells can be mature or immature. Immature pancreatic islet hormone-expressing cells can be distinguished from mature pancreatic islet hormone-expressing cells based on the differential expression of certain markers, or based on their functional capabilities, e.g., glucose responsiveness.

In certain embodiments of the present invention, the terms "enriched", "isolated", "separated", "sorted", "purified" or purifying by depleting or equivalents thereof refer to a cell culture or a cell population or cell sample that contains at least 15%, 20%, 25%, 30%, 35%, 40%, 45%, 50%, 55%, 60%, 65%, 70%, 75%, 80%, 85%, 90%, 95%, 96%, 97%, 98%, 99% or 100% of the desired cell lineage or a desired cell having a certain cell phenotype, e.g., expressing a certain cell marker or not expressing a certain cell marker gene characteristic of that cell phenotype. Methods for purifying, enriching, isolating, separating, sorting, and/or depleting endoderm lineage cells derived from hES cells are also described in U.S. Application Number 12/107,020, entitled METHODS FOR PURIFYING ENDODERM AND PANCREATIC ENDODERM CELLS DERIVED FORM HUMAN EMBRYONIC STEM CELLS, filed April 21, 2008..

Many stem cell media culture or growth environments are embodied in the present invention, including defined media, conditioned media, feeder-free media, serum-free media and the like. As used herein, the term "growth environment" or "milieu" or equivalents thereof is an environment in which undifferentiated or differentiated stem cells (e. g., primate embryonic stem cells) will proliferate in vitro. Features of the environment include the medium in which the cells are cultured, and a supporting structure (such as a substrate on a solid surface) if present. Methods for culturing or maintaining hES cells and/or differentiating hES cells are also described in PCT/US2007/062755 entitled Compositions and methods useful for culturing differentiable cells, filed February 23, 2007; U.S. Application Number 11/993,399, entitled Embryonic stem cell culture compositions and methods of use thereof, filed December 20, 2007; and U.S. Application Number 11/875,057, entitled Methods and compositions for feeder-free pluripotent stem cell media containing human serum, filed October 19, 2007.

As used herein, the term "defined medium" or "defined media" or equivalents thereof refers to a biochemically defined formulation comprised solely of the biochemically-defined constituents. A defined medium can include solely constituents having known chemical compositions. A defined medium can also include constituents that are derived from known sources. For example, a defined medium can also include factors and other compositions secreted from known tissues or cells; however, the defined medium will not include the conditioned medium from a culture of such cells. Thus, a "defined medium" can, if indicated, include particular compounds added to form the culture medium. Defined media compositions are known in the art, for example, PCT/US2007/062755 filed June 13, 2007 and marketed as StemPro®hESC SFM by Invitrogen, Carlsbad, California.

As used herein, the phrase "conditioned medium" refers to a medium that is altered as compared to a base medium. For example, the conditioning of a medium can cause molecules, such as nutrients and/or growth factors, to be added to or depleted from the original levels found in the base medium. In some embodiments, a medium is conditioned by allowing cells of certain types to be grown or maintained in the medium under certain conditions for a certain period of time. For example, a medium can be conditioned by allowing hESCs to be expanded, differentiated or maintained in a medium of defined composition at a defined temperature for a defined number of hours. As will be appreciated by those of skill in the art, numerous combinations of cells, media types, durations and environmental conditions can be used to produce nearly an infinite array of conditioned media.

As used herein, the term "feeder cells" or "feeder cell layers" are cells which grow in vitro and are co-cultured with a target cell, e.g., co-cultured with stem cells. As used herein, the term "essentially free of a feeder cell" or "feeder-free" and equivalents thereof, refer to tissue culture conditions that do not contain intentionally added feeder cells. Also, a cell culture is "essentially feeder-free" when it does not contain exogenously added conditioned medium taken from a culture of feeder cells nor exogenously added feeder cells in the culture, where "no exogenously added feeder cells" means that cells to develop a feeder cell layer have not been purposely introduced for that reason. Of course, if the cells to be cultured are derived from a seed culture that contained feeder cells, the incidental co-isolation and subsequent introduction into another culture of some small proportion of those feeder cells along with the desired cells (e. g., undifferentiated primate stem cells) should not be deemed as an intentional introduction of feeder cells. In such an instance, the culture contains a de minimus number of feeder cells. By "de minimus", it is meant that number of feeder cells that are carried over to the instant culture conditions from previous culture conditions where the differentiable cells can have been cultured on feeder cells. Similarly, feeder cells or feeder-like cells that develop from stem cells seeded into the culture shall not be deemed to have been purposely introduced into the culture.

As used herein, the term "basal medium" refers to a solution of amino acids, vitamins, salts, and nutrients that is effective to support the growth of cells in culture, although normally these compounds will not support cell growth unless supplemented with additional compounds. The nutrients include a carbon source (e.g., a sugar such as glucose) that can be metabolized by the cells, as well as other compounds necessary for the cells' survival. These are compounds that the cells themselves cannot synthesize, due to the absence of one or more of the gene(s) that encode the protein(s) necessary to synthesize the compound (e.g., essential amino acids) or, with respect to compounds which the cells can synthesize, because of their particular developmental state the gene(s) encoding the necessary biosynthetic proteins are not being expressed as sufficient levels. A number of base media are known in the art of mammalian cell culture, such as Dulbecco's Modified Eagle Media (DMEM), Knockout-DMEM (KO-DMEM), and DMEM/F12, although any base medium that supports the growth of primate embryonic stem cells in a substantially undifferentiated state can be employed. A "basal medium" as described herein also refers to the basal medium described in PCT/US2007/062755, filed June 13, 2007.

As used herein, the term "enzymatically dissociated" or "enzymatically treated" or equivalents thereof refer to dissociating clusters or embryoid bodies of hES cells or hES-derived cells into single cell populations. Because passaging of hES cells as clusters imposes some limitations for downstream applications requiring single cell preparations, e.g., cell separation by flow cytometry (FACS) and/or dispensing of live hES cells into multi-well plates for use in high throughput screening. Cowan et al., describe enzymatic dissociation of hES cells using trypsin but they are propagated as small clusters rather than single cells. See Cowan et al., New Engl. J. Med. 350:1353-1356, 2004; and Hasegawa et al., Stem Cells 24:2649-2660, 2006. In one embodiment of the invention, hES cells or hES-derived cells are enzymatically treated or dissociated using Accutase. Dissociated cells can be grown to a high density as monolayers, and still retain their pluripotency, in the case of undifferentiated hES cells. Use of Accutase is also described in PCT/US2007/62755, entitled COMPOSITIONS AND METHODS USEFUL FOR CULTURING DIFFERENTIABLE CELLS, filed February 23, 2007.

The basal medium may or may not contain "exogenous insulin or insulin substitutes", which refers to insulin or insulin substitutes that is/are not intentionally added to the compositions or methods of the embodiments disclosed herein. Thus, in certain embodiments, the methods and compositions are free of insulin or insulin substitutes that are intentionally supplied. The compositions or methods may, however, not necessarily be free of endogenous insulin. As used herein, "endogenous insulin" indicates that the cultured cells may be producing insulin of their own accord when cultured according to the methods of the present invention. Endogenous insulin also can be used to indicate residual impurities from the primary cell culture or impurities from the starting materials. In specific examples, the compositions and/or methods of the described herein include less than about 5 µg/ml, 4 µg/ml, 3 µg/ml, 2 µg/ml, 1 µg/ml, 0.5 µg/ml, 0.25 µg/ml or 0.1 µg/ml of insulin. In other examples, the compositions and/or methods described herein include from about 0.7 ng/ml to about 7 ng/ml of insulin or less than 0.7 ng/ml of insulin. In still other examples, the compositions and/or methods described herein include, insubstantial amounts of insulin, less than insubstantial amounts of insulin or no detectable amounts of insulin. Insulin detection can be performed using methods commonly known in the art. Methods for maintaining hES cells or differentiating hES cells in a media containing insulin or lack thereof are also described in U.S. Application Number 11/360,167, entitled Compositions and methods for differentiation of human embryonic stem cells utilizing insulin or IGF treatment, filed February 8, 2006.

Moreover, it has been demonstrated that high insulin concentration, e.g., concentrations as little as 0.2 µg/ml, is detrimental for the production of definitive endoderm cells from hES cells. See McLean et al. Stem Cells 25:29-38 2007. Contacting the cells with insulin during the TGF-beta stimulated differentiation process keeps the cells in the pluripotent undifferentiated state (Step 1 of D'Amour et al. 2006, supra). Hence, presence of high insulin can promote production of other hES-derived cells types other than definitive endoderm, which production is necessary for the further production of other endodermally-derived, hES-derived, cell types described herein. Production of hES-derived cells in a culture medium containing Knockout serum (GIBCO BRL) or StemPro34 (GIBCO BRL) supplement contains about 8-16 µg/ml and 15 µg/ml, respectively. See U.S. Publication 2006/0003446 to Keller et al. In contrast, hES-derived cells produced herein, in particular production of definitive endoderm cells, lack substantial amounts of insulin, e.g., the ranges described herein and in the related applications indicated above, describe production of definitive endoderm in a culture having about 0.7ng to 7ng/ml of insulin.

To be clear, the term "insulin" refers to the protein, or variant or fragment thereof that binds to the insulin receptor in normal physiological concentrations and can induce signaling through the insulin receptor. The term "insulin" encompasses a protein having the polypeptide sequence of native human insulin, or of other mammalian insulin, or of any homologs or variants to these sequences. Additionally, the term insulin encompasses polypeptide fragments that are capable of binding to the insulin receptor to induce signaling through the insulin receptor. The term "insulin substitute" refers to any zinc containing compound that can be used in place of insulin to give substantially similar results as insulin. Examples of insulin substitutes include, but are not limited to, zinc chloride, zinc nitrate, zinc bromide, and zinc sulfate.

Insulin-like growth factors are not insulin substitutes or homologs of insulin, as contemplated in the embodiments presented herein. Accordingly, in another specific embodiment, the compositions and methods of the present invention comprise the use of at least one insulin-like growth factor (IGF) or a variant or a functional fragment thereof. In another embodiment, the compositions and methods of the present invention are free of any exogenous insulin-like growth factors (IGFs). In specific embodiments, the compositions and methods of the present invention contain about, less than, less than about, no greater than or no greater than about 200, 150, 100, 75, 50, 25, 20, 15, 10, 9, 8, 7, 6, 5, 4, 3, 2, or 1 ng/ml of IGF-1.

The culture conditions of embodiments described herein are "isotonic," which term refers to a solution having essentially the same tonicity (i.e., effective osmotic pressure equivalent) as another solution with which it is compared. In the context of cell culture, an "isotonic" medium is one in which cells can be cultured without an appreciable net flow of water across the cell membranes.

When used in connection with cell cultures and/or cell populations, the term "portion" means any non-zero amount of the cell culture or cell population, which ranges from a single cell to the entirety of the cell culture or cells population. In preferred embodiments, the term "portion" means at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20%, at least 21%, at least 22%, at least 23%, at least 24%, at least 25%, at least 26%, at least 27%, at least 28%, at least 29%, at least 30%, at least 31%, at least 32%, at least 33%, at least 34%, at least 35%, at least 36%, at least 37%, at least 38%, at least 39%, at least 40%, at least 41%, at least 42%, at least 43%, at least 44%, at least 45%, at least 46%, at least 47%, at least 48%, at least 49%, at least 50%, at least 51%, at least 52%, at least 53%, at least 54%, at least 55%, at least 56%, at least 57%, at least 58%, at least 59%, at least 60%, at least 61%, at least 62%, at least 63%, at least 64%, at least 65%, at least 66%, at least 67%, at least 68%, at least 69%, at least 70%, at least 71%, at least 72%, at least 73%, at least 74%, at least 75%, at least 76%, at least 77%, at least 78%, at least 79%, at least 80%, at least 81%, at least 82%, at least 83%, at least 84%; at least 85%, at least 86%, at least 87%, at least 88%, at least 89%, at least 90%, at least 91%, at least 92%, at least 93%, at least 94% or at least 95% of the cell culture or cell population.

With respect to cells in cell cultures or in cell populations, the term "substantially free of" or "essentially free of" means that the specified cell type of which the cell culture or cell population is free, is present in an amount of less than about 10%, less than about 9%, less than about 8%, less than about 7%, less than about 6%, less than about 5%, less than about 4%, less than about 3%, less than about 2% or less than about 1% of the total number of cells present in the cell culture or cell population.

As used herein, "marker", or equivalents thereof refers to any molecule that can be observed or detected. For example, a marker can include, but is not limited to, a nucleic acid, such as a transcript of a specific gene, a polypeptide product of a gene, a non-gene product polypeptide, a glycoprotein, a carbohydrate, a glycolipid, a lipid, a lipoprotein or a small molecule (for example, molecules having a molecular weight of less than 10,000 amu).

As used herein, "ligand" refers to a moiety or binding partner that specifically binds or cross-reacts to the marker or target or receptor or membrane protein on the cell or to the soluble analyte in a sample or solution. The target on the cell, includes but is not limited to a marker. Ligands can also include an antibody that binds to a cellular antigen, an antibody that binds a soluble antigen, an antigen that binds an antibody already bound to the cellular or soluble antigen; or fragments of such antibodies and antigens that are capable of binding; a nucleic acid sequence sufficiently complementary to a target nucleic acid sequence of the cellular target or soluble analyte to bind the target or analyte sequence, a nucleic acid sequence sufficiently complementary to a ligand nucleic acid sequence already bound to the cellular marker or target or soluble analyte, or a chemical or proteinaceous compound, such as biotin or avidin. Ligands can be soluble or can be immobilized on the capture medium (i.e., synthetically covalently linked to a bead), as indicated by the assay format, e.g., antibody affinity chromatography. As defined herein, ligands include various agents that detect and react with one or more specific cellular markers or targets or soluble analytes. Further, all such ligands are characterized by the desired ability to bind the specified marker or target or analyte, whether it is soluble or bound to a cell. In one preferred embodiment, the ligand of the invention is a component that preferentially binds to all or a portion of a cell surface receptor. Thus, a ligand useful in this embodiment of the invention can be an antibody or a functional fragment thereof capable of binding to a cell surface receptor on a hES or hES-derived population.

As used herein, "sufficient amount," "amount sufficient" or grammatical equivalents means an amount or concentration of a substance to achieve a desired effect. For example, in some embodiments, a sufficient amount can refer to an amount or concentration of a substance that causes at least a portion of the cells in a cell population or cell culture to differentiate into cells of a desired type. A sufficient amount can be expressed as a concentration, such as at least 0.1 ng/ml, at least 0.5 µg/ml, at least 1 ng/ml, at least 2 ng/ml, at least 5 ng/ml, at least 10 ng/ml, at least 15 ng/ml, at least 20 ng/ml, at least 25 ng/ml, at least 30 ng/ml, at least 35 ng/ml, at least 40 ng/ml, at least 45 µg/ml, at least 50 ng/ml, at least 55 ng/ml, at least 60 µg/ml, at least 65 ng/ml, at least 70 ng/ml, at least 75 ng/ml, at least 80 ng/ml, at least 85 ng/ml, at least 90 ng/ml, at least 95 ng/ml, at least 1000 ng/ml, at least 200 ng/ml, at least 300 ng/ml, at least 400 ng/ml, or at least 500 ng/ml. In some embodiments, the concentration may be expressed in a range, such as from 1 ng/ml to 100 ng/ml or any subrange contained therein, for example, from 10 ng/ml to 50 ng/ml. In other embodiments, sufficient amount is expressed in terms of the effect achieved. In such embodiments, a sufficient amount can be an amount that is sufficient to produce a population or culture of cells containing at least 10% at least 15%, at least 20%. at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98% or at least 99% of a desired cell type from a population or culture of cells containing 0% or a lesser percent of cells of the desired type.

In one embodiment, ligands described herein are members of the TGF-beta super family, As used herein, "TGF-beta superfamily member" or equivalents thereof refers to over 30 structurally related proteins including subfamilies including TGF-beta1, TGF-beta2, TF-beta3, GDF-15, GDF-9, BMP-15, BMP-16, BMP-3, GDF-10, BMP-9, BMP-10, GDF-6, GDF-5, GDF-7, BMP-5, BMP-6, BMP-7, BMP-8, BMP-2, BMP-4, GDF-3, GDF-1, GDF 11, GDF8, Activins betaC, betaE, betaA and betaB, BMP-14, GDF-14, MIS, Inhibin alpha, Lefty1, Lefty2, GDNF, Neurteurin, Persephin and Artemin. See Chang et al. (2002) Endocrine Rev. 23(6):787-823. These ligands are synthesized as prepropeptides of approximately 400-500 amino acids (aa). The N-terminal variable length pro-region is cleaved at a consensus RXXR site prior to secretion. The secreted C-terminal mature segment has spatially conserved cysteines that form a cysteine knot structure in the monomer. It is the conserved dimeric structure that provide specificity for receptor binding and biological function. Small secondary structural elements arising from the non-conserved regions give family members their specificity for ligand-receptor binding. See Souchelnytskyi, S. et al. (2002) Trends Cell Biol. 12:304; Massague, J. & Y.-G. Chen (2000) Genes Dev. 14:627; Zhou, X. et al. (1993) Nature 361:543; and Kumar, A. et al. (2001) J. Biol. Chem. 276:656.

A TGF-beta family member can be replaced by, or used in conjunction with, a TGF-beta signaling pathway activator, which is a compound that stimulates one or more of the polypeptides or interactions that participate in transducing or otherwise effectuating changes in the properties of a cell in response to a TGF-beta family member. A TGF-beta signaling pathway includes TGF-beta family members themselves. TGF-beta super family members transmit signals to the nucleus by signaling through type II and I serine-threonine kinase receptors and intracellular effectors known as Smads. These receptors fall into two subfamilies known as type I and type II receptors that act cooperatively to bind ligand and transduce signal (Attisano et al., Mol Cell Biol 16 (3), 1066-1073 (1996)). Ligands bind to type I and II receptors on the cell surface, promoting activation of the type I receptor via phosphorylation. This activated complex in turn activates intracellular Smads, which assemble multi-subunit complexes that regulate transcription. Members of the TGF-beta super family are divided into two signaling subgroups: those functionally related to TGF-beta/Activin and those related to the BMP/GDF subfamily. Most TGF-beta ligands are thought to bind first to a type II receptor and this ligand/type II receptor complex then recruits or phosphorylates a type I receptor (Mathews, L S, Endocr Rev 15:310-325 (1994); Massague, Nature Rev: Mol Cell Biol. 1, 169-178 (2000)). The type II receptor kinase by phosphorylating and activating the type I receptor kinase, which then phosphorylates and activates the Smad proteins. The TGF-beta/Activin ligands bind to TGF-beta and Activin type II receptors and can activate Smad-2 and -3. Nodal and Lefty signal through this Activin-type pathway. The BMP/GDF ligands bind to BMP type II receptors and can activate Smads 1, 5, and 8. See Derynck, R et al. Cell 95, 737-740 (1998)). Upon ligand stimulation, Smads move into the nucleus and function as components of transcription complexes.

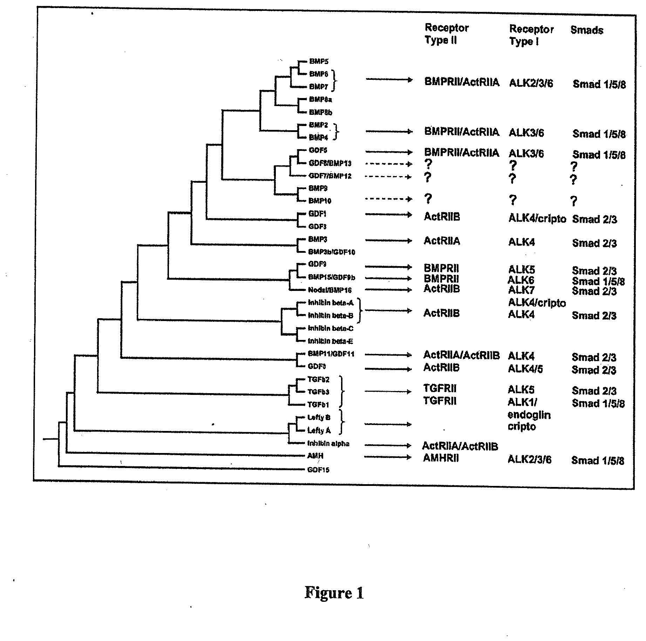

In one embodiment described herein, GDF8 and GDF11, a TGF-beta family member is also a TGF-beta signaling pathway activator by binding to the extracellular ligand binding domain portion of the ActRII receptor and then forming a complex with ActRI, leading to the inhibition of the Smad7 negative regulator and phosphorylation of the Smad2/Smad3 complex. The Smad2/Smad3 complex associates with Smad4 to regulate expression of certain genes. See FIG.1, adapted from Mazerbourg et al. (2005) Biol Chem 280(37): 32122-32132.

TGF-beta signaling is regulated positively and negatively though various mechanisms. Positive regulation amplifies signals to a level sufficient for biological activity. TGF-beta superfamily ligands bind to a type II receptor, which recruits and phosphorylates a type I receptor. The type I receptor then phosphorylates receptor-regulated SMADs (R-SMADs e.g., SMAD1, SMAD2, SMAD3, SMAD5, and SMAD8) which can now bind common mediator Smad or co-SMAD. R-SMAD/coSMAD complexes accumulate in the nucleus where they act as transcription factors and participate in the regulation of target gene expression.

Negative regulation of TGF-beta signaling occurs at the extracellular, membrane, cytoplasmic and nuclear levels. Signal transduction can be interrupted in order to repress signaling initiated by TGF-beta. This can be accomplished by any means known in the art in which the interaction between the TGF-beta receptor(s) and the SMAD protein is antagonized or prevented, including proteins that block or compete with TGF-beta receptor and SMAD protein interactions. Alternatively, the transcription or translation of TGF-beta receptor or SMAD protein can be altered by any means known in the art in order to prevent signal transmission along the signaling pathway. Positive and negative regulation of various TGF-beta member proteins is further exemplified by a more detail description of some of the factors below.

Examples of Growth differentiation factors include 1, 2, 3, 5, 6, 7, 8, 9, 10, 11, and 15. Growth differentiation factor-1 (GDF1) is a TGF-beta family member that has a role in left-right patterning and mesoderm induction during embryonic development. It is found in the brain, spinal cord and peripheral nerves of embryos. See Rankin et al. (2000) Nat Genet 24 (3): 262-5; Lee S. (1991) Proc Natl Acad Sci U S A 88 (10): 4250-4; and Rankin et al. (2000) Nat Genet 24 (3): 262-5. The physiological receptor of GDF1 is thought to be activin receptor-like kinase 4. See Cheng, et al. (2003) Genes Dev. 17, 31-36.

Another TGF-beta family member is GDF2, a protein also known as bone morphogenetic protein (BMP)-9. GDF2 has a role in inducing and maintaining the ability of embryonic basal forebrain cholinergic neurons to respond to a neurotransmitter called acetylcholine; basal forebrain cholinergic neurons are important for the processes of learning, memory and attention. GDF2 is also important for the maturation of basal forebrain cholinergic neurons. The physiological receptor of GDF2 is thought to be ALK1 (also called ACVRL1), an endothelial-specific type I receptor of the TGF-beta receptor family. See Lopez-Coviella et al. (2005) Proc Natl Acad Sci U S A 102 (19): 6984-9; and Truksa et al. (2006) Proc Natl Acad Sci U S A 103 (27): 10289-93. GDF2 is also a potent BMP to induce orthotopic bone formation in vivo. See Kang et al. (2004) Gene Therapy 11 (17): 1312-20.

In one embodiment of the invention, GDF3 is used in the differentiation methods described herein. GDF3 is also known as Vg-related gene 2 (Vgr-2). GDF-3 is significantly similar to other TGF-β superfamily members called Vgl (found in frogs), BMP2, BMP6 and GDF1, but lacks one of the conserved cysteine residues common to all other TGF-β superfamily members. See Caricasole et al. (1998) Oncogene 16 (1): 95-103. GDF-3 is a bifunctional protein that inhibits some TGF-β superfamily members (e.g. BMPs) while activating others, thus regulating the balance between different modes of TGF-beta signaling. It has been shown to negatively and positively control differentiation of embryonic stem cells in mice and humans. See Levine A, Brivanlou A (2006) Cell Cycle 5 (10): 1069-73; and (2006). Development 133 (2): 209-16. This molecule plays a role in mesoderm and definitive endoderm formation during the pre-gastrulation stages of development. See Chen et al. (2006) Development 133 (2): 319-29. GDF3 is thought to interact with the same type of receptors and activate the same SMADs as GDF1. See Cheng, et al. (2003) supra.

Growth differentiation factor 5 (cartilage-derived morphogenetic protein-1), also known as GDF5, is a human gene. The protein encoded by this gene is a member of the bone morphogenetic protein (BMP) family and the TGF-beta super family. This group of proteins is characterized by a polybasic proteolytic processing site which is cleaved to produce a mature protein containing seven conserved cysteine residues. The members of this family are regulators of cell growth and differentiation in both embryonic and adult tissues. GDF5 is expressed in the developing central nervous system, and has a role in skeletal and joint development. See O'Keeffe et al. (2004) J Neurocytol 33 (5): 479-88; and Buxton et al. (2001) J Bone Joint Surg Am 83-A Suppl 1 (Pt 1): S23-30. The physiological receptor of GDF5 is thought to be BMPRII/ActRIIA & ALK3/6, which in turn activates SMAD1/5/8 proteins. See Nishitoh et al. (1996) J. Biol. Chem. 271: 21345-21352; and Cheng, et al. (2003) supra.

Still another, TGF-beta member is GDF6. GDF6 can regulate patterning of the ectoderm by interacting with bone morphogenetic proteins, and control eye development. See Chang et al. (1999Development 126 (15): 3347-57; and Asai-Coakwell et al. (2007) Am J Hum Genet 80 (2): 306-15. GDF7 is expressed in the roof plate that is located in the developing nervous system of embryos. The roof plate is required for the generation of several classes of spinal cord dorsal interneuron. GDF7 specifically induces the formation of sensory neurons in the dorsal spinal cord from neural crest cells by generating signals within the roof plate. See Lo et al. (2005) Proc Natl Acad Sci U S A 102 (20): 7192-7; and Lee et al. (1998) Genes Dev 12 (21): 3394-407. The physiological mechanisms of GDF6 and GDF7 are unclear and/or not known. See Cheng, et al. (2003) supra.

Another TGF-beta family member, GDF8, also referred to as myostatin, is expressed for the most part in the cells of developing and adult skeletal muscle tissue. Myostatin appears to play an essential role in negatively controlling skeletal muscle growth (McPherron et al. Nature (London) 387, 83-90 (1997)). Antagonizing myostatin has been shown to increase lean muscle mass in animals (McFerron et al, supra, Zimmers et al, Science 296:1486 (2002)). GDF8 is synthesized as an inactive preproprotein complex approximately 375 amino acids in length (GenBank Accession No: AAB86694 human sequence) and is activated by proteolytic cleavage at a tetrabasic processing site to produce an N-terminal inactive prodomain and an approximately 109 amino acid C-terminal protein which dimerizes to form a homodimer of about 25 kDa. This homodimer is the mature, biologically active protein (Zimmers et al., Science 296, 1486 (2002)). So, as used herein, the term "prodomain" or "propeptide" refers to the inactive N-terminal protein which is cleaved off to release the active C-terminal protein. As used herein the term "myostatin" or "mature myostatin" refers to the mature, biologically active C-terminal polypeptide, in monomer, dimer or other form, as well as biologically active fragments or related polypeptides including allelic variants, splice variants, and fusion peptides and polypeptides. The mature myostatin has been reported to have 100% sequence identity among many species including human, mouse, chicken, porcine, turkey, and rat (Lee et al., PNAS 98, 9306 (2001)). The physiological receptor for GDF8 is thought to be ActRIIB & ALK4/5, which in turn activates SMAD 2/3 proteins. See Cheng, et al. (2003) supra.

Growth differentiation factor 9 (GDF9), is a human gene and expressed in oocytes and is thought to be required for ovarian folliculogenesis. GDF9 also plays an important role in the development of primary follicles in the ovary. It has a critical role in granulosa cell and theca cell growth, as well as in differentiation and maturation of the oocyte. See et al. (2002) J Clin Endocrinol Metab 87 (1): 316-21. The cell surface receptor through which GDF9 generates a signal is the bone morphogenetic protein type II receptor (BMPR2). See Vitt et al. (2002) Biol Reprod 67 (2): 473-80. The physiological receptor for GDF9 is thought to be BMPRII & ALK5, which in turn activates SMAD 2/3 proteins. The physiological receptor for a variant of GDF9, GDF9b, is the same except instead of ALK5, it thought to be ALK6. See Cheng, et al. (2003) supra.

GDF10 is closely related to bone morphogenetic protein-3 (BMP3), and plays a role in head formation and can have multiple roles in skeletal morphogenesis. GDF10 is also known as BMP-3b, with GDF10 and BMP3 regarded as a separate subgroup within the TGF-beta superfamily. In mice, GDF10 mRNA is abundant in the brain, inner ear, uterus, prostate, neural tissues, blood vessels and adipose tissue with low expression in spleen and liver. It is also present in bone of both adults and neonatal mice. Human GDF10 mRNA is found in the cochlea and lung of fetuses, and in testis, retina, pineal gland, and other neural tissues of adults. See Cunningham et al. (1995) Growth Factors 12 (2): 99-109; and Katoh (2006) Int J Mol Med 17 (5): 951-5. The physiological receptor for GDF10 is thought to be ActRIIA & ALK4, which in turn activates SMAD 2/3 proteins. See Cheng, et al. (2003) supra.

Another TGF-beta family is GDF11, which has approximately 90% identity to myostatin at the amino acid level. GDF-11 is involved in the regulation of anterior/posterior patterning of the axial skeleton (McPherron et al, Natr Genet 22 (93): 260-264 (1999); Gamer et al, Dev. Biol. 208 (1), 222-232 (1999)) but postnatal functions are unknown. Thus, as used herein, members of the "GDF-8/GDF-11 subfamily" are those polypeptide(s) (or an encoding nucleic acid) comprising an amino acid sequence that is at least 50%, 60%, 60%, 80%, 90%, or 100% identical to an amino acid sequence of a mature, naturally occurring GDF8 or GDF11 polypeptide, such as the mature human or mouse GDF8 and GDF11 amino acid sequences, or at least 50%, 60%, 60%, 80%, 90%, or 100% identical to functional fragments thereof. A member of the GDF-8/GDF-11 subfamily should also retain the ability to stimulate a receptor-mediated signaling pathway. The physiological receptor for GDF11 is thought to be ActRIIA/ActRIIB & ALK4, which in turn activates SMAD 2/3 proteins. See Cheng, et al. (2003) supra.

In addition, there is GDF15, which has a role in regulating inflammatory and apoptotic pathways in injured tissues and during disease processes. GDF15 is also known as TGF-PL, MIC-1, PDF, PLAB, and PTGFB. GDF15 mRNA is most abundant in the liver, with lower levels seen in some other tissues. Its expression in liver can be significantly up-regulated in during injury of organs such as liver, kidney, heart and lung. See Zimmers et al. (2005) Shock 23 (6): 543-8. Hsiao et al. (2000) Mol Cell Biol 20 (10): 3742-51; and Ago et al. (2006) Circ Res 98 (3): 294-297.

Anti-Müllerian hormone (AMH) is a dimeric glycoprotein that inhibits the development of the Müllerian ducts in a male embryo. It has also been called Müllerian inhibiting factor (MIF), Mullerian inhibiting hormone (MIH), and Mullerian inhibiting substance (MIS). AMH is a protein hormone structurally related to inhibin and activin, and a member of the transforming growth factor-β (TGF-β) family. It is present in fish, reptiles, birds, marsupials, and placental mammals. See Weenen et al. (2004) Mol Hum Reprod 10 (2): 77-83.

Inhibins (e.g., inhibins A, B, C & E) inhibit FSH synthesis and secretion, and participates in the regulation of the menstrual cycle. Inhibin contains an alpha and beta subunit linked by disulfide bonds. Two forms of inhibin differ in their beta subunits (A or B), while their alpha subunits are identical. Activin is a related peptide that counteracts inhibin. See van Zonneveld et al. (2003) Hum Reprod 18 (3): 495-501; Skinner et al. (1989) Mol Cell Endocrinol 66 (2): 239-49; Robertson et al. (2004) Mol Cell Endocrinol 225 (1-2): 65-71. These family members are involved in the regulation of a wide range of biological processes including cell proliferation, differentiation, and other functions. Activins were originally discovered as gonadal peptides involved in the regulation of follicle stimulating hormone (FSH) synthesis, and are now believed to be involved in a variety of regulatory and development function including, early embryonic development. Activins A, B and AB are the homodimers and heterdimer respectively of two polypeptide chains, BA and BB (Vale et al. Nature 321, 776-779 (1986), Ling et al., Nature 321, 779-782 (1986)). These two polypeptide chains can also dimerize with a related a chain giving rise to inhibins A and B respectively (Mason et al, Nature 318, 659-663 (1986)). The physiological receptor for inhibins/activins is ActRIIB & ALK4, which in turn activates SMAD 2/3 proteins. See Cheng, et al. (2003) supra.

Nodal is a distant member of the TGF-beta super family, sharing only 25-39% amino acid identity to other superfamily members in the mature region. It was first identified for its role in specification of mesodermal and endodermal cell fates in the mouse, and named for its strong expression in the node (a dorsal mesoderm organizing center that gives rise to the dorsal midline structures). The physiological receptor for Nodal is ActRIIB & ALK4/cripto and ALK7, which in turn activates SMAD 2/3 proteins. See Cheng, et al. (2003) supra. Nodal signaling is also mediated by the EGF-CFC family of cofactors (e.g., cripto), which are not involved in Activin signaling. See Reissmann (Aug 2001) Genes Dev. 15 (15); Kumar, A. et al. (2001) J. Biol. Chem. 276:656; Shen, M.M. & A.F. Schier (2000) Trends Genet. 16:303; and Schier, A.F. & M.M. Shen (1999) Nature 403:385.

Transforming growth factor beta (TGF-beta) is a protein that comes in three isoforms called TGF-betal, TGF-beta2 and TGF-beta3. TGF-betal, TGF-beta2, and TGF-beta3 all function through the same receptor signaling systems. TGF-beta controls proliferation, cellular differentiation, and other functions in most cell types. It can also act as a negative autocrine growth factor. TGF-beta acts synergistically with TGF-alpha in inducing cellular transformation. Specific receptors for TGF-beta activation trigger apoptosis when activated. Many cells synthesize TGF-beta and almost all of them have specific receptors for this peptide. The peptide structures of the three members of the TGF-beta family are highly similar and all are encoded as large protein precursors (TGF-beta1 contains 390 amino acids and TGF-beta2 and TGF-beta3 each contain 412 amino acids). They each have an N-terminal signal peptide of 20-30 amino acids that they require for secretion from a cell, a pro-region (called latency associated peptide or LAP), and a 112-114 amino acid C-terminal region that becomes the mature TGF-beta molecule following its release from the pro-region by proteolytic cleavage. See Khalil N (1999) Microbes Infect 1 (15): 1255-63. The mature TGF-beta protein dimerizes to produce a 25 KDa active molecule with many conserved structural motifs. See Herpin et al. (2004) Dev Comp Immunol 28 (5): 461-85. TGF-beta has nine cysteine residues that are conserved among its family; eight form disulphide bonds within the molecule to create a cysteine knot structure characteristic of the TGF-beta superfamily while the ninth cysteine forms a bond with the ninth cysteine of another TGF-beta molecule to produce the dimer. See Daopin et al. (1992) Science 257 (5068): 369-73. Many other conserved residues in TGF-beta are thought to form secondary structure through hydrophobic interactions. The region between the fifth and sixth conserved cysteines houses the most divergent area of TGF-beta molecules that is exposed at the surface of the molecule and is implicated in receptor binding and specificity of TGF-beta. The physiological receptor for TGF-betal, TGF-beta2 and TGF-beta3 bind to either the TGFRII & ALK1/endoglin cripto, which in turn activates SMAD 1/5/8 proteins; or they activate the ALK5 receptor which in turn activates SMAD 2/3 proteins. See Cheng, et al. (2003) supra.

Ligands which are "TGF-beta family member" are also intended to include single polypeptides and homo- and hetero-dimers or other multimeric forms, variant forms of any of the naturally occurring polypeptides, including fusion proteins, truncated proteins, mutant forms, as well as nucleic acids encoding polypeptides that are TGF-beta family members, and any functional fragment thereof that retains the ability to stimulate a receptor-mediated signaling pathway and further have sufficient sequence similarity to a naturally occurring family member as to be recognizable using a sequence comparison algorithm such as BLAST or PILEUP. The subject ligands can be agonist (e.g. mimics), or alternatively, an antagonist of a biological TGF-beta signaling activity of, e.g., the polypeptide is able to modulate differentiation and/or growth and/or survival of a cell responsive to the subject ligand. Homologs of the subject ligand include versions of the protein which are resistant to post-translation modification, as for example, due to mutations which alter modification sites (such as tyrosine, threonine, serine or aspargine residues), or which inactivate an enzymatic activity associated with the protein. The subject ligands can also be provided as chimeric molecules, such as in the form of fusion proteins. For instance, the ligand can be provided as a recombinant fusion protein which includes a second polypeptide portion, e.g., a second polypeptide having an amino acid sequence unrelated (heterologous) to the ligand, e.g. the second polypeptide portion is glutathione-S-transferase, e.g. the second polypeptide portion is an enzymatic activity such as alkaline phosphatase, e.g. the second polypeptide portion is an epitope tag.

Still in another embodiment, ligands include molecules which activate the Wnt signaling pathway, which is essential for mesoderm formation. The Wnt genes belong to a family of proto-oncogenes and encode over 20 cysteine-rich secreted glycoproteins that activate the Wnt signaling pathway by binding to Frizzled (Fz) receptors found on target cells. Binding of Wnt ligands to Fz receptors activates the Dishevelled (Dsh/Dv11) protein, allowing it to inhibit the activity of the multiprotein complex comprising beta-catenin, axin-adenomatous polyposis coli (APC) and glycogen synthase kinase (GSK)-3beta. Inhibition of the beta-catenin/APC/GSK-3beta complex prevents phosphorylation of beta-catenin by GSK-3beta. Phosphorylated beta-catenin is targeted for ubiquitin mediated degradation by the proteosome. Therefore, Wnt binding to the Fz receptor results in beta-catenin accumulation in the cytoplasm. See Bakre et al. (2007) J Biol Chem. 282(43):31703-31712. Wnt signaling is detected in pregastrulating embryo, through primitive streak (PS) formation, and during gastrulation. See Cordenonsi et al., (2003) Proc. Natl. Acad. Sci. U. S. A. 100, 3299-3304. Mice lacking the ligand Wnt3, beta-catenin, or the receptors Lrp5/6 do not form PS or mesoderm. See Kelly et al., (2004) Development (Camb.) 131, 2803-2815. In ES cells, the stability of beta-catenin, the operative molecule of the canonical Wnt pathway, is controlled by glycogen synthase kinase 3β (GSK-3beta) via phosphorylation and subsequent degradation. Upon Wnt pathway activation, GSK-3beta is inhibited, and the non-phosphorylated beta-catenin is stabilized and enters the nucleus to activate transcription of Wnt-regulated genes. See Gregorieff et al., (2005) Genes Dev. 19, 877-890. Pharmacological inhibitors of GSK-3beta have been used as direct intracellular activators of the canonical Wnt pathway. GSK-3beta inhibitors (also referred to herein as GSK3 inhibitors) include, but are not limited to, commercially available GSK-3beta inhibitors, for example, those inhibitors available through EMD Biosciences, Madison, Wisconsin, including 1-Azakepaullone, Aloisine, Alsterpaullone, FRATtide (188-225 amino acids of FRAT1), GSK-3beta Inhibitor No. IX, X, XIII, XIV, XV, and GSK-3beta Inhibitor I, II, III, VI, VII, VIII, IX, XI, XII, Peptide Inhibitor (Cat.361545 and 361546), Indirubin-3'-monoxime, Indirubin-3'-monoxime 5-lodo, Indirubin-3'-monoxime-5-sulfonic Acid, and Kenpaullone and functional derivatives thereof. Also, other known GSK-3beta inhibitors include small molecules such as lithium, bivalent zinc, beryllium, aloisines, hymenialdisine, indirubins, maleimides, muscarinic agonists, pyridazinone derivatives, e.g., pyrazolo[3,4-b]quinoxalines, 5-aryl-pyrazolo[3,4-b]pyridazines, and functional derivatives thereof.

In a preferred embodiment, the compositions and/or methods described herein include a GSK3 inhibitor that is a pyridocarbazolo-cyclopentadienyl ruthenium complex. In especially preferred embodiments this inhibitor is GSK3 inhibitor XV (also referred to as GSK3 inhibitor number 15). The structure of GSK3 inhibitor number XV has the following structure:

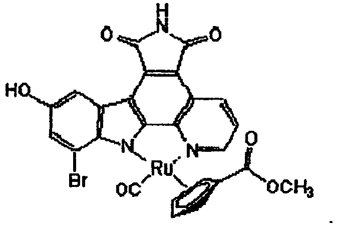

If other preferred embodiments, the compositions and/or methods described herein include dihydropyrimidine inhibitors such as those described by Ring et al. (2003) Diabetes 52: 588-95.
Of this class, two inhibitors, CHIR98014 and CHIR99021 are especially preferred. The structure of GSK3 inhibitor CHIR99021, which is exemplary of this class, is as follows:

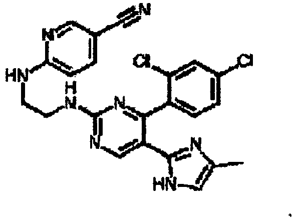

As used herein, the term "percent identical" refers to sequence identity between two sequences, e.g. amino acid or nucleotide sequences. Percent identity can be determined by comparing a position in each sequence which can be aligned for purposes of comparison. Expression as a percentage of identity refers to a function of the number of identical amino acids or nucleic acids at positions shared by the compared sequences. Various alignment algorithms and/or programs can be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md.

For most markers or targets described herein, the official Human Genome Organization (HUGO) gene symbol is provided. Such symbols, which are developed by the HUGO Gene Nomenclature Committee, provide unique abbreviations for each of the named human genes and gene products. These gene symbols are readily recognized and can easily be associated with a corresponding unique human gene and/or protein sequence by those of ordinary skill in the art.

In accordance with the HUGO designations, the following gene symbols are defined as follows: PPIG - cyclophilin G; TBP - TATA-box binding protein; GHRL - ghrelin; IAPP - islet amyloid polypeptide; INS - insulin; GCG - glucagon; ISL1 - islet-1 transcription factor, PAX6 - paired box gene 6; PAX4 - paired box gene 4; NEUROG3 - neurogenin 3 (NGN3); NKX2-2 - NKX2 transcription factor related, locus 2 (NKX2.2); NKX6-1 - NKX6 transcription factor related, locus 1 (NKX6.1); IPF1 - insulin promoter factor 1 (PDX1); ONECUT1 - one cut domain, family member 1 (HNF6); HLXB9 - homeobox B9 (HB9); TCF2 - transcription factor 2, hepatic (HNF1b); FOXA1- forkhead box A1 (HNF3alpha); FOXA2-forkhead box A2 (HNF3beta); HGF - hepatocyte growth factor; IGF1 - insulin-like growth factor 1; POU5F1 - POU domain, class 5, transcription factor 1 (OCT4); NANOG - Nanog homeobox; SOX2 - SRY (sex determining region Y)-box 2; CDH1 - cadherin 1, type 1, E-cadherin (ECAD); T - brachyury homolog (BRACH); FGF4 - fibroblast growth factor 4; WNT3 - wingless-type MMTV integration site family, member 3; SOX17 - SRY (sex determining region Y)-box 17; GSC - goosecoid; CER1 - cerberus 1, cysteine knot superfamily, homolog (CER); CXCR4 - chemokine (C-X-C motif) receptor 4; FGF17 - fibroblast growth factor 17; SOX7 - SRY (sex determining region Y)-box 7; SOX1 - SRY (sex determining region Y)-box 1; AFP - alpha-fetoprotein; SPARC - secreted protein, acidic, cysteine-rich (osteonectin); THBD - thrombomodulin (TM); CDX2 - caudal type homeobox 2; NCAM - neural cell adhesion molecule; ZIC1 - Zic family member 1; NEF3 - neurofilament 3 (NFM); SST - somatostatin; MAFA - v-maf musculoaponeurotic fibrosarcoma oncogene homolog A; MAFB - v-maf musculoaponeurotic fibrosarcoma oncogene homolog B; SYP - synaptophysin; CHGA - chromogranin A (parathyroid secretory protein 1).

The following provides the full gene names corresponding to non-HUGO gene symbols as well as other abbreviations that can be used herein: SS - somatostatin (SOM); PP - pancreatic polypeptide; C-peptide - connecting peptide; Ex4 - exendin 4; NIC - nicotinamide and DAPT - N-[N-(3,5-difluorophenacetyl)-L-alanyl]-S-phenylglycine t-butyl ester; RA - retinoic acid; RPMI - Roswell Park Memorial Institute medium; CMRL - Connaught Medical Research Labs medium; FBS - fetal bovine serum; NBP10 - NCAM binding protein 10; PTF1A-pancreas specific transcription factor la. The terms fibroblast growth factor 7 (FGF7) and keratinocyte growth factor (KGF) are synonymous.

The features and other details of the invention, either as steps of the invention or as combinations of parts of the invention, will now be more particularly described. It will be understood that the particular embodiments of the invention are shown by way of illustration and not as limitations of the invention. The principle features of this invention can be employed in various embodiments without departing from the scope of the invention.

### Human Embryonic Stem Cells and hES-derived Cell Types

With respect to certain embodiments of the present invention, the differentiation culture conditions and hES-derived cell types described herein are substantially similar to that described in D'Amour et al. 2006. D'Amour et al. 2006 describe a 5 step differentiation protocol: stage 1 (definitive endoderm), stage 2 (primitive gut tube or foregut endoderm), stage 3 (posterior foregut or PDX1 endoderm), stage 4 (pancreatic endoderm, or pancreatic epithelium and endocrine precursor) and stage 5 (hormone expressing endocrine cell).

A preferred method for deriving these hES-derived cell types utilizes human embryonic stem cells as the starting material. Disclosed herein are pluripotent cells that can be cells that originate from the morula, embryonic inner cell mass or those obtained from embryonic gonadal ridges. Human embryonic stem cells can be maintained in culture in a pluripotent state without substantial differentiation using methods that are known in the art. Such methods are described, for example, in US Patent Nos. 5,453,357, 5,670,372, 5,690,926 5,843,780, 6,200,806 and 6,251,671.

In some processes, hESCs are maintained on a feeder layer or can be maintained feed-free. In such processes where a feeder layer is employed, any feeder layer which allows hESCs to be maintained in a pluripotent state can be used. One commonly used feeder layer for the cultivation of human embryonic stem cells is a layer of mouse fibroblasts. More recently, human fibroblast feeder layers have been developed for use in the cultivation of hESCs (see U.S. Patent Application No. 10/486,408, filed February 6, 2004, and entitled Alternative Compositions & Methods for the Culture of Stem Cells.

Alternative processes permit the maintenance of pluripotent hESC without the use of a feeder layer. Methods of maintaining pluripotent hESCs under feeder-free conditions are also described in PCT/US2007/062755, filed February 23, 2007, entitled Compositions and methods useful for culturing differentiable cells; and U.S. Patent Application No. 11/875,057, filed October 19, 2007 entitled METHODS AND COMPOSITIONS FOR FEEDER FREE PLURIPOTENT STEM CELL MEDIA CONTAINING HUMAN SERUM.

The human embryonic stem cells used herein can be maintained in culture either with or without serum ("serum-free"). In some embryonic stem cell maintenance procedures, serum replacement is used. In others, serum free culture techniques, such as those described in U.S. Patent Application No. 2003/0190748 are used. PCT/US2007/062755, filed February 23, 2007, entitled Compositions and methods useful for culturing differentiable cells also describes serum-free culture conditions. Stem cells are maintained in culture in a pluripotent state by routine passage until it is desired that they be differentiated into lineages of the three germ layers, including endodermal lineages, further including definitive endoderm, foregut endoderm, PDX1 foregut endoderm, pancreatic epithelium, endocrine precursor cells and/or pancreatic islet hormone-expressing or endocrine cells. Method of differentiating pluripotent hESCs to these cells types are described in related applications as indicated above and substantially as described in D'Amour et al. 2005 and 2006, supra.

### Monitoring of hES-derived Cells

The developmental progression of the hES-derived cells described herein (e.g., cells produced as a result of Stages or Steps 1-5 as described in D'Amour et al. 2006. supra) can be monitored by determining the expression of markers characteristic of each hES-derived cell type along the developmental pathway. In some processes, the identification and characterization of a hES-derived cell type is by expression of a certain marker or different expression levels and patterns of more than one marker. That is, the presence or absence, the high or low expression, of one or more the marker(s) typifies and identifies a cell-type. Also, certain markers can have transient expression, whereby the marker is highly expressed during one stage of development and poorly expressed in another stage of development. The expression of certain markers can be determined by measuring the level at which the marker is present in the cells of the cell culture or cell population as compared to a standardized or normalized control marker. In such processes, the measurement of marker expression can be qualitative or quantitative. One method of quantitating the expression of markers that are produced by marker genes is through the use of quantitative PCR (Q-PCR). Methods of performing Q-PCR are well known in the art.

In some embodiments of the present invention, the presence, absence and/or level of expression of a marker is determined by quantitative PCR (Q-PCR). For example, the amount of transcript produced by certain genetic markers, such as SOX17, CXCR4, OCT4, AFP, TM, SPARC, SOX7, CDX2, MIXL1, GATA4, HNF3beta, HNF4alha, GSC, FGF17, VWF, CALCR, FOXQ1, CMKOR1, CRIP1 and other markers described herein is determined by quantitative Q-PCR. In other embodiments, immunohistochemistry is used to detect the proteins expressed by the above-mentioned genes. In still other embodiments, Q-PCR can be used in conjunction with immunohistochemical techniques or flow cytometry techniques to effectively and accurately characterize and identify cell types and determine both the amount and relative proportions of such markers in a subject cell type. In one embodiment, Q-PCR can quantify levels of RNA expression in a cell culture containing a mixed population of cells. However, Q-PCR cannot provide or qualify whether the subject markers or proteins are co-expressed on the same cell. In another embodiment, Q-PCR is used in conjunction with flow cytometry methods to characterize and identify cell types. Thus, by using a combination of the methods described herein, and such as those described above, complete characterization and identification of various cell types, including endoderm lineage type cells, can be accomplished and demonstrated.

Still, other methods which are known in the art can also be used to quantitate marker gene expression. For example, the expression of a marker gene product can be detected by using antibodies specific for the marker gene product of interest (e.g., e.g. Western blot, flow cytometry analysis, and the like). In certain processes, the expression of marker genes characteristic of hES-derived cells as well as the lack of significant expression of marker genes characteristic of hES-derived cells. Still further methods for characterizing and identifying hES-derived cells types are described in related applications as indicated above.

The expression of tissue-specific gene products can also be detected at the mRNA level by Northern blot analysis, dot-blot hybridization analysis, or by reverse transcriptase initiated polymerase chain reaction (RT-PCR) using sequence-specific primers in standard amplification methods. See U.S. Pat. No. 5,843,780 for further details. Sequence data for particular markers listed in this disclosure can be obtained from public databases such as GenBank.

The choice of primers for use in nucleic acid amplification will depend on the marker or target nucleic acid sequence. Primers used in the embodiments described herein are generally oligonucleotides, usually deoxyribonucleotides several nucleotides in length, that can be extended in a template-specific manner by the polymerase chain reaction. The design of suitable primers for amplifying a marker or target nucleic acid is within the skill of practitioners in the art. In general, the following factors are considered in primer design: a) each individual primer of a pair preferably does not self-hybridize; b) the individual pairs preferably do not cross-hybridize; and c) the selected pair must have the appropriate length and sequence homology in order to anneal to two distinct regions flanking the nucleic acid segment to be amplified. However, not every nucleotide of the primer must anneal to the template for extension to occur. The primer sequence need not reflect the exact sequence of the marker or target nucleic acid. For example, a non-complementary nucleotide fragment can be attached to the 5' end of the primer with the remainder of the primer sequence being complementary to the target. Alternatively, non-complementary bases can be interspersed into the primer, provided that the primer sequence has sufficient complementarily with the target for annealing to occur and allow synthesis of a complementary nucleic acid strand.

For a convenient detection of the amplified nucleotide acids resulting from PCR or any other nucleic acid amplification reactions described above or known in the art, primers can be conjugated to a detectable label. Detectable labels suitable for use in the present invention include any composition detectable by spectroscopic, photochemical, biochemical, immunochemical, electrical, optical or chemical means. A wide variety of appropriate detectable labels are known in the art, which include luminescent labels, enzymatic or other ligands. In preferred embodiments, one will likely desire to employ a fluorescent label or an enzyme tag, such as digoxigenin, β-galactosidase, urease, alkaline phosphatase or peroxidase, avidin/biotin complex.

Amplification primer combinations suitable for use in amplification assays include the following: PPIG (GenBank NM_004792): primers CTTGTCAATGGCCAACAGAGG (SEQ. ID NO:1); GCCCATCTAAATGAGGAGTTGGT (SEQ. ID NO:2); TBP (GenBank NM_003194): primers TGTGCACAGGAGCCAAGAGT (SEQ. ID NO:3); ATTTTCTTGCTGCCAGTCTGG (SEQ. ID NO:4). INS (GenBank NM_000207): primers AAGAGGCCATCAAGCAGATCA (SEQ. ID NO:5); CAGGAGGCGCATCCACA (SEQ. ID NO:6). NKX6-1 (NM_006168): primers CTGGCCTGTACCCCTCATCA (SEQ. ID NO:7); CTTCCCGTCTTTGTCCAACAA (SEQ. ID NO:8). PDX1 (NM_000209): primers AAGTCTACCAAAGCTCACGCG (SEQ. ID NO:9); GTAGGCGCCGCCTGC (SEQ. ID NO:10). NEUROG3 (NM_020999): primers GCTCATCGCTCTCTATTCTTTTGC (SEQ. ID NO:11); GGTTGAGGCGTCATCCTTTCT (SEQ. ID NO:12). FOXA2 (NM_021784): primers GGGAGCGGTGAAGATGGA (SEQ. ID NO:13); TCATGTTGCTCACGGAGGAGTA (SEQ. ID NO:14). GCG (NM_002054): primers AAGCATTTACTTTGTGGCTGGATT (SEQ. ID NO:15); TGATCTGGATTTCTCCTCTGTGTCT (SEQ. ID NO:16). ONECUT (NM_030712): primers CGCTCCGCTTAGCAGCAT (SEQ. ID NO:17); GTGTTGCCTCTATCCTTCCCAT (SEQ. ID NO:18). HNF4A (NM_000457): primers GAAGAAGGAAGCCGTCCAGA (SEQ. ID NO:19), GACCTTCGAGTGCTGATCCG (SEQ. ID NO:20). SOX17 (NM_022454): primers GGCGCAGCAGAATCCAGA (SEQ. ID NO:21); CCACGACTTGCCCAGCAT (SEQ. ID NO:22). HLXB9 (NM_005515): primers CACCGCGGGCATGATC (SEQ. ID NO:23); ACTTCCCCAGGAGGTTCGA (SEQ. ID NO:24). NKX2-2 (NM_002509): primers GGCCTTCAGTACTCCCTGCA (SEQ. ID NO:25); GGGACTTGGAGCTTGAGTCCT (SEQ. ID NO:26). PTF1A (NM_178161): primers GAAGGTCATCATCTGCCATCG (SEQ ID NO:27) GGCCATAATCAGGGTCGCT (SEQ ID NO:28). SST (NM_001048): primers CCCCAGACTCCGTCAGTTTC (SEQ ID NO:29); TCCGTCTGGTTGGGTTCAG (SEQ ID NO:30). PAX6 (NM_000280): primers CCAGAAAGGATGCCTCATAAAGG (SEQ ID NO:31); TCTGCGCGCCCCTAGTTA (SEQ ID NO:32). CDX2 (NM_00126S): primers GGGCTCTCTGAGAGGCAGGT (SEQ ID NO:33); CCTTTGCTCTGCGGTTCTG (SEQ ID NO:34); and SOX7 (NM_031439): ACGCCGAGCTCAGCAAGAT (SEQ ID NO:35); TCCACGTACGGCCTCTTCTG (SEQ ID NO:36).

In other embodiments, the expression levels of any marker in hES-derived cell types or hES-derived cell populations is at least about 2-fold higher, at least about 4-fold higher, at least about 6-fold higher, at least about 8-fold higher, at least about 10-fold higher, at least about 15-fold higher, at least about 20-fold higher, at least about 40-fold higher, at least about 80-fold higher, at least about 100-fold higher, at least about 150-fold higher, at least about 200-fold higher, at least about 500-fold higher, at least about 750-fold higher, at least about 1000-fold higher, at least about 2500-fold higher, at least about 5000-fold higher, at least about 7500-fold higher or at least about 10,000-fold higher as compared to a standardized or normalized control marker.

### Methods for identifying agents for production of endoderm

Certain embodiments described herein provide for methods of assessing the effectiveness of a test agent and/or ligand for production or induction of endoderm cells from hES cells. Such methods can be used to screen libraries of test agents. In general, a method of this type involves assessing the ability of the test agent and/or ligand to interfere with or promote TGF-beta pathway signaling. For example, screening assays include but are not limited to biochemical and cell-based assays.

Biochemical assays typically contact a test agent and/or ligand with a known TGF-beta pathway activator polypeptide. An agent that binds can potentially activate or inhibit signaling. Test agents can be assessed in an assay system that measures binding between a TGF-beta family member, such as a GDF8/GDF11 family member, and a receptor, such as an ActRIIA or ActRIIB.

In a further embodiment, a cell based assay contacts an hES cell culture with the test agent and/or ligand, and an activity of a TGF-beta signaling pathway is detected, whereby a test agent that increases the activity of a TGF-beta signaling pathway has an increased likelihood of being effective for increasing endoderm production.

Yet in another embodiment, described herein are methods for screening various compounds for their ability to modulate or induce hES cells, such as, for example, by affecting growth, proliferation, maturation or differentiation, or by endoderm production. In an illustrative embodiment, the subject cells can be used to screen various compounds or natural products, such as small molecules or growth factors. Such compounds can be tested for essentially any effect, with exemplary effects being cell proliferation or differentiation. The efficacy of the compound can be assessed by generating dose response curves from data obtained using various concentrations of the compound. A control assay can also be performed to provide a baseline for comparison. Identification of the progenitor cell population(s) amplified in response to a given test agent and/or ligand can be carried out according to such phenotyping as described above. Assays such as those described above can be carried out *in vitro* (e.g. with cells in culture) or *in vivo* (e.g. with cell implanted in a subject).

### Kits

The conventional reagents for high throughput assays or other diagnostic assays useful according to the disclosure can be provided in the form of kits. In the disclosure a kit is provided for performance of the above-described methods. Preferably such kits are employed for performing the diagnostic methods of the disclosure and/or monitoring therapy. However, in the disclosure, such kits can also be assembled for research purposes. Thus, in the disclosure, a kit contains the components taught above, e.g., at least one soluble ligand that binds a cellular target in the sample; at least one soluble ligand that binds a soluble analyte in the sample or at least one competing soluble analyte (preferably labeled); and a solid phase capture medium that binds directly to the soluble analyte, indirectly to the soluble analyte, or to the soluble ligand that binds to the soluble analyte. In the disclosure, the kits can include instructions for performing the particular assay, various diluents and buffers, and/or signal-generating reagents, such as fluorophores, enzyme substrates, cofactors and chromogens. Other components can include indicator charts for colorimetric comparisons, disposable gloves, decontamination instructions, applicator sticks or containers, and/or a sample preparator cup.

In the disclosure, a kit useful for the performance of the above-described immuno assays include, as a component, a solid matrix or a solid phase capture medium associated with multiple first ligands that bind the cellular marker or target and is associated with a first detectable label. The kit can further comprise a second, third and/or fourth ligand that is capable of binding to a second, third and/or fourth cellular marker or target and or soluble analyze. The second, third and/or fourth ligands are associated with a second, third and/or fourth detectable label.

In the disclosure, a kit for performing another of the competitive inhibition assays described above, contains a first ligand associated with a first label. Multiple first ligands are capable of binding to a single cellular target. Another component is a competing analyte associated with a second label. Still another component is the solid phase capture medium on which are immobilized multiple of ligands or antibodies capable of binding to the soluble analyte (either competing soluble analyte or soluble analyte naturally occurring in the sample).

In the disclosure, a kit for performing the immune complex assay described herein includes a first ligand capable of binding to a first cellular target and providing a first detectable signal; a second ligand capable of binding to the soluble analyte and providing a second detectable signal; a third ligand capable of binding to the same soluble analyte; a solid phase capture medium on which is immobilized multiple fourth ligands, the fourth ligands capable of binding to the third ligands.

In the disclosure, such kits are useful for evaluating hES populations and/or hES-derived cell population samples for purposes of enriching, isolating, depleting, separating, sorting, purifying and/or determining levels of certain cell types or bound components or soluble antigens or analytes thereof. Such a diagnostic kit contains the dyes, ligands, capture medium, and other components of the methods of this disclosure. Such kits also contain labels, exemplified above, pre-attached to the other components of the specific assay to be performed, or provided separately for attachment to a selected component, e.g., a substrate. Alternatively, such kits can contain a simple mixture of such compositions or means for preparing a simple mixture.

### EXAMPLES

Having generally described this invention, a further understanding can be obtained by reference to certain specific examples which are provided herein for purposes of illustration only, and are not intended to be limiting.

### Reference EXAMPLE 1

### Directed Differentiation of Pluripotent Stem Cells to DEFINITIVE Endoderm Cells Using GDF8 or GDF11

The following reference example describes at least two TGF-beta member growth factors that can efficiently differentiate hES cells to definitive endoderm lineage cells.

Human embryonic stem (hES) cells were differentiated in vitro to definitive endoderm (stage 1) for about 2-4 days substantially as described in D'Amour et al. 2006 Nat Biotechnol 24(11):1392-401 and U.S. Patent Application Publication Number 2007/0154984, which are herein incorporated in their entireties. Briefly, undifferentiated human embryonic stem

(hES) cells were maintained on mouse embryo fibroblast feeder layers (Millipore, formerly Chemicon or Specialty Media) or on human serum coated plates (Valley Biomedical) in DMEM/F12 (Mediatech) supplemented with 20% KnockOut serum replacement (Invitrogen/Gibco), 1 mM nonessential amino acids (Invitrogen/Gibco), Glutamax (Invitrogen/Gibco), penicillin/streptomycin (Invitrogen/ Gibco), 0.55 mM 2-mercaptoethanol (Invitrogen/Gibco) and 4 ng/ml to 20ng/mL recombinant human FGF2 (R&D Systems). Low dosages of Activin A (10-25 ng/ml, R&D Systems) were added to the growth culture medium to help maintain undifferentiated growth. Cultures were either manually passaged, or enzymatically passaged using 5ug/mL dispase (Invitrogen/Gibco), or passaged using Accutase (Innovative Cell Technologies #AT104) at 1:4-1:15 split ratios every 3-7 days. Before initiating differentiation, hES cells were given a brief wash in PBS+/+ (PBS+/+ refers to PBS containing both calcuim and magnesium) (Mediatech).

Four hES cell culture plates (CyT49; passage 41) were then differentiated in RPMI (Mediatech) supplemented with Glutamax, penicillin/streptomycin, and with either: i) 100 ng/mL activin A, ii) 100 ng/mL of activin B (R&D Systems), iii) 100 ng/mL of GDF8, or iv) 100 ng/mL of GDF11 (R&D Systems) and varying concentrations of defined FBS (HyClone). Additionally, 0.1% BSA (Invitrogen/Gibco) and 75ng/mL Wnt3a (R&D Systems) were added on the first day (d0) of differentiation. FBS concentrations were 0% for the first 24 h and 0.2% for the second 24 h. Replicate samples were collected from each culture condition at 0, 24 and 48 hours after differentiation commenced and analyzed for relative gene expression using real-time quantitative PCR.

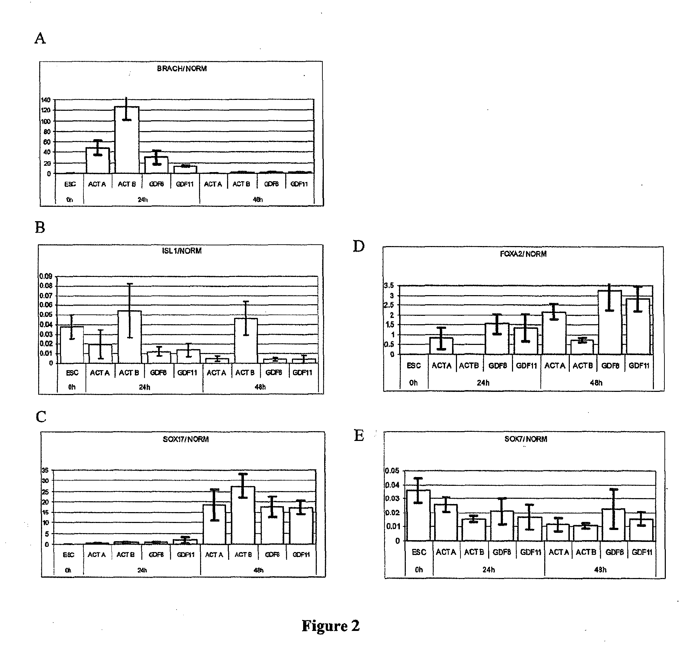

As shown in FIG. 2, induction of definitive endoderm differentiation from hES cells was accomplished by using sufficient amounts of GDF8 and/or GDF11. The addition of 100 ng/ml of GDF8 resulted in a 1324-fold induction of SOX17 (FIG.2C) and 438-fold induction of FOXA2 (FIG.2D) gene expression, as compared to undifferentiated hESCs. The addition of 100 ng/ml of GDF11 resulted in a 1306-fold induction of SOX17 (FIG.2C) and a 382-fold induction of FOXA2 (FIG.2D) by day 2 of differentiation, as compared to undifferentiated hESCs. In addition, the differentiation to definitive endoderm proceeded through a mesendoderm intermediate as evidenced by the transient brachyury expression witnessed at 24 hours (FIG.2A). The lack of induction of SOX7 (FIG.2E) gene expression indicated that the endoderm lineage produced was definitive endoderm and not extra-embryonic endoderm, which does appreciably express SOX7. The higher expression of ISL1 (FIG.2B) observed in the activin B treated cultures as compared to activin A treated cultures, indicated that the activin B induced a higher proportion of mesoderm differentiation. It further demonstrated that both GDF8 alone and GDF11 alone was as effective as activin A for induction of definitive endoderm differentiation derived from hES cells. The lower FOXA2 (FIG.2D) induction in the activin B treated cultures was also indicative of the enhanced mesoderm differentiation induced by activin B treatment.

These data indicate that sufficient amounts of GDF8 and/or GDF11 are efficient in generating definitive endoderm cell populations as compared to Activin A and more efficient compared to activin B.

### Reference EXAMPLE 2

### Directed Differentiation of Definitive Endoderm Cells to Successive Endoderm Intermediate Type Cells Using GDF8 or GDF11

Human ES cell cultures were maintained and differentiated substantially as described above in reference Example 1, and further differentiated to foregut endoderm (stage 2) and then onto pancreatic endoderm and pancreatic endocrine cells (stages 3 and 4). After 48 hours of differentiation to definitive endoderm (stage 1) the cells were briefly washed in PBS+/+ and then further differentiated in RPMI supplemented with 0.2% FBS, Insulin-Transferrin-Selenium (1:1000), Glutamax, penicillin/streptomycin, and 25ng/mL KGF (R&D Systems) for 3 days. On the first day of stage 2, the culture also received TGF-beta inhibitor No. 4 (3-(6-Methylpyridin-2-yl)-4-(4-quinolyl)-1-phenylthiocarbamoyl-1H-pyrazole) at 2.5 uM (EMD Bioscience). After three days in stage 2 the cells were then differentiated for one day in DMEM with B27 supplement (1:100) containing all-trans retinoic acid (RA) at 0.2 uM and KAAD-cyclopamine at 0.25 uM. Following the single day of retinoid exposure, the cells were cultured in DMEM with B27 (1:100) containing noggin at 40 ng/ml. At day 9 of total differentiation, replicate samples were collected from each culture condition and analyzed for relative gene expression using real-time quantitative PCR.

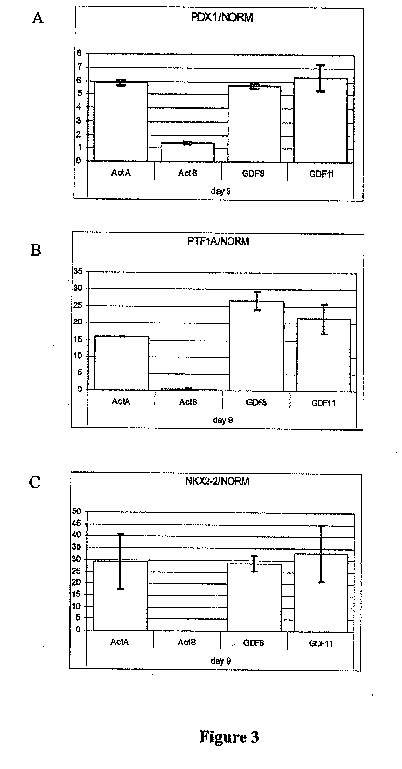

As shown in FIG.3, definitive endoderm produced using sufficient amounts of GDF8 and/or GDF11 were further differentiated to pancreatic endoderm and pancreatic endocrine cells. Subsequent differentiation of definitive endoderm to pancreatic endoderm expressing PDX1 and PTF1A (FIG.3A-B), or endocrine cells expressing NKX2-2 (FIG.3C), was as effective in the GDF8 or GDF11 conditions as it was for activin A produced definitive endoderm. The cultures of definitive endoderm produced using activin B were less effective at further differentiating to pancreatic lineages. These results were unexpected given that GDF8 and GDF11 are about 80% identical in the mature regions of the protein. However, as these results indicate, the effectiveness of any growth or differentiation factor may not be anticipated or predicted based on the homology of the factors alone, i.e., effectiveness and efficiency of the factors must often be determined experimentally or empirically.

### Reference EXAMPLE 3

### Directed Differentiation of Pluripotent Stem Cells Using Other TGF-Beta Family Member Proteins

Undifferentiated human embryonic stem (hES) cells (CyT49; passage 48) were maintained substantially as described in reference Example 1 above. Seven hES cell culture plates were then differentiated in RPMI (Mediatech) supplemented with Glutamax, penicillin/streptomycin, and either: i) activin A (100 ng/ml), ii) TGF-beta 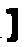 (100 ng/ml), iii) TGF-beta3 (100 ng/mL), iv) TGF-beta2 (200 ng/ml), v) GDF3 (500 ng/ml), vi) GDF9 (4 ug/ml), or vii) GDF15 (2.5 ug/ml) and varying concentrations of defined FBS (HyClone). Additionally, 0.1% BSA (Invitrogen/Gibco) and 75ng/mL Wnt3a (R&D Systems) were added on the first day (d0) of differentiation. FBS concentrations were 0% for the first 24 h and 0.2% for the second 24 h. Replicate samples were collected from each culture condition at 0, 24 and 48 hours after differentiation commenced and analyzed for relative gene expression using real-time quantitative PCR.

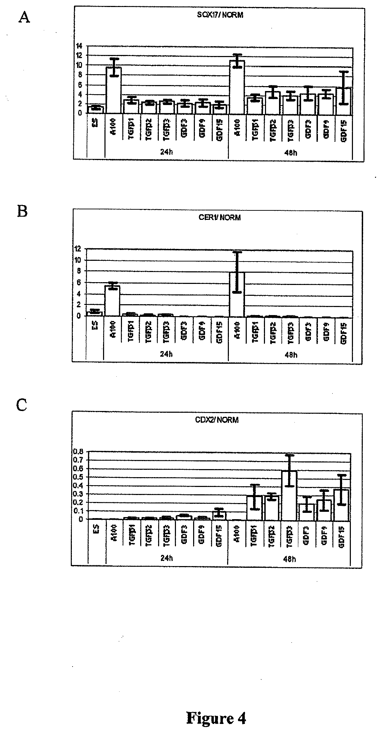

As shown in FIG.4, definitive endoderm was produced only in the activin A treatment condition (A100) as indicated by the induction of SOX17 (FIG.4A) and CER1 (FIG.4B) gene expression. Conversely, treatment with any of the other TGF-beta family member ligands resulted in little to no induction of SOX17 or CER1 but instead resulted in substantially induced expression of CXD2 (FIG.4C) over both the hES and the definitive endoderm expression levels. The induction levels for CDX2 observed in non-activin treated plates (i.e. TGF-beta 1, 2, 3 or GDF3, GDF9 and GDF15) over that observed with the activin A treatment was in the range from 900-fold higher expression for GDF3 to 2,667-fold higher expression for TGF-beta3. These data indicated that the described TGF-beta family ligands were not effective for directing differentiation of hES cells to definitive endoderm, at least under conditions similar to those where activin A was effective. This further highlights that the effectiveness of differentiation factors may not be anticipated or predicted based on the homology of the factors (FIG.1) to each other alone, or just because they are members in a family of factors, rather effectiveness and efficiency of the factors must often be determined experimentally or empirically.

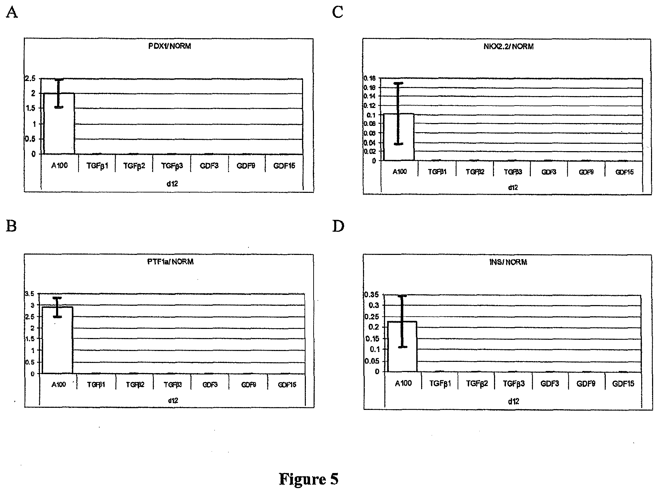

To further demonstrate that the described TGF-beta family ligands were not sufficient to produce effective definitive endoderm differentiation, the plates were subjected to conditions that promote efficient differentiation of definitive endoderm cells to pancreatic endoderm, and subsequently endocrine cells. Such conditions are described in Example 2 above. In this example, however, the stage 4 noggin treatment lasted for 6 days and replicate samples were taken for real-time PCR analysis at a total of 12 days of differentiation as compared to 9 days of differentiation in Example 2. As shown in FIGs.5A-D, pancreatic endoderm and endocrine cell types were produced only in the activin A treatment condition (A100) as indicated by the induction of PDX1, PTF1A, NKX2-2 and INS gene expression. Pancreatic endoderm or endocrine type cells had PDX1 expression levels ranging between 1413-fold and 14,497-fold higher in the presence of activin A, as compared to in the presence of TGF-betal and GDF15, respectively (FIG.5A). Similarly, the pancreatic endoderm or endocrine cells expressing endocrine markers NKX2-2 and INS were typically not detected in the non-activin A treated plates (FIG.5C-D). Stated another way, the activin A treated plates were 1200-fold or more higher in expression of these markers than the non-Activin A treated plates.

### Reference EXAMPLE 4

### Directed Differentiation of Pluripotent Stem Cells to Definitive Endoderm Cells Using Wnt3a or GSK3-Beta Inhibitors

Undifferentiated hES cells (CyT49; passage 35) were maintained substantially as described in reference Example 1 above. Ten hES cell culture plates were then differentiated in RPMI (Mediatech) supplemented with Glutamax, penicillin/streptomycin, and varying concentrations of defined FBS (HyClone). FBS concentrations were 0% for the first 24 h and 0.2% for the second 24 h. Additionally, 0.1% BSA (Invitrogen/Gibco) was added on the first day only. All plates received 100 ng/mL activin A for both days. In addition, but on the first day only, plates received either Wnt3a (R&D Systems) at 25, 50 or 75 ng/mL, or GSK3 inhibitor No. 9 ("BIO") (EMD Bioscience) at 10 or 100 nM, or GSK3 inhibitor No. 15 (EMD Bioscience) at 1, 10 or 100 nM, or GSK3 inhibitor No. 8 (EMD Bioscience) at 10 or 100 nM. Replicate samples were collected from each culture condition at 48 hours after differentiation commenced and analyzed for relative gene expression using real-time quantitative PCR.

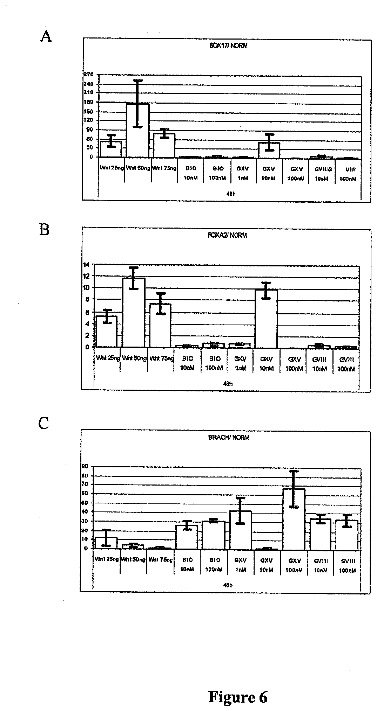

As shown in FIG. 6, the expression levels of SOX17 and FOXA2 at 48 hours of differentiation were substantially higher in the Wnt3a differentiation conditions and the condition that received 10 nM of GSK3 inhibitor No.15 (GXV) as compared to the other GSK inhibitors (FIG.6A-B). Neither GSK3 inhibitor No. 9 ("BIO") nor GSK3 inhibitor No. 8 (GVIII) at 10 or 100 nM resulted in substantial differentiation of hES cells to definitive endoderm (FIG.6A-B). Reduction in definitive endoderm production was also indicated by the relatively higher levels of brachyury gene expression, which levels of expression are not observed in definitive endoderm cells. In addition, the use of GSK3 inhibitor No. 15 was dose-sensitive as evidenced by the much poorer definitive endoderm production using either 1 or 100 nM as compared to 10 nM (FIG.A-C).

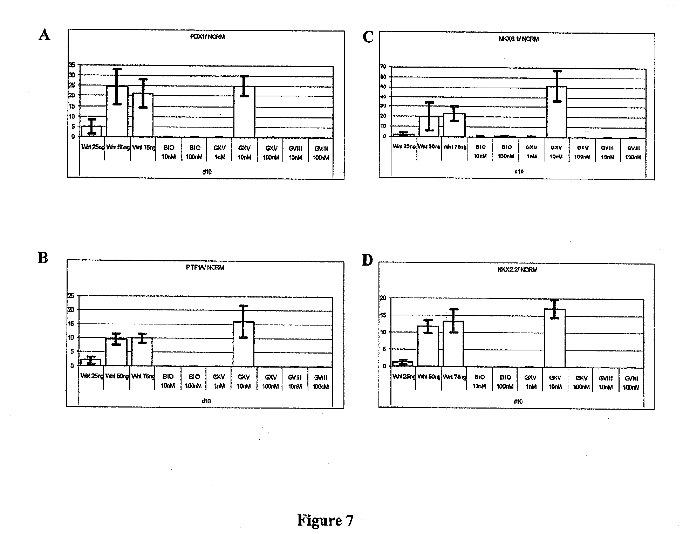

GSK3 inhibitor No.15 (GXV) was more effective for production of definitive endoderm than GSK3 inhibitor No. 9 ("BIO") and No.8 (GVIII). To further demonstrate this, plates treated with the inhibitors were further differentiated to pancreatic endoderm and endocrine cells using the methods substantially as described in Example 2 above. However, in this instance, the stage 4 noggin treatment lasted for 4 days, as compared to 3 days in Example 2, and replicate samples were taken for real-time PCR analysis at a total of 10 days of differentiation, as compared to 9 days in Example 2. As indicated by the induction of PDX1, PTF1A, NKX6-1 and NKX2-2 gene expression in FIG. 7, pancreatic endoderm and endocrine cell types were produced in much greater abundance when definitive endoderm was produced using Wnt3a at 50 or 75 ng/ml and with GXV at 10 nM. PDX1 expression levels ranged between 1,046-fold and 3,701-fold higher in the GXV (10 nM) treatment condition than for BIO and GVIII, respectively. Similarly, the expression of the endocrine marker NKX2-2 was 1,721-fold and 4,784-fold higher in the GSK3 inhibitor No.15 (GXV; 10 nM) treatment condition than for GSK3 inhibitor No. 9 (BIO) and GSK3 inhibitor No. 8 (GVIII), respectively.

Taken together, these data indicate the GSK3 inhibitor No.15 (GXV) can effectively replace Wnt3a when used in combination with activin A for the differentiation of hES cells to definitive endoderm. The results again suggest that not all GSK3 inhibitors function equivalently and that the effectiveness of small molecules as effectors of hES cell differentiation may not be anticipated or predicted based on their described activity alone, but must often be determined experimentally and empirically.

### Reference EXAMPLE 5

### Directed Differentiation of Pluripotent Stem Cells to Definitive Endoderm Cells Expressing CXCR4 Using Activin A or GDF8 in Combination with Wnt3a or a GSK3-Beta Inhibitor

Undifferentiated hES cells (BG02; passage 32) were maintained substantially as described in reference Example 1 above. Four hES cell culture plates were then differentiated in RPMI (Mediatech) supplemented with Glutamax, penicillin/streptomycin, and varying concentrations of defined FBS (HyClone). FBS concentrations were 0% for the first 24 h and 0.2% for the second and third days. Growth factors used on the first day of differentiation were either 100 ng/mL activin A or 100 ng/mL of GDF8 (R&D Systems) in combination with either 75 ng/mL Wnt3a (R&D Systems) or 10 nM GSK3 inhibitor No. 15 (GXV; EMD Bioscience). Additionally, 0.1% BSA (Invitrogen/Gibco) was added on the first day only. During days 2 and 3 only the activin A or GDF8 were added (i.e., no Wnt3a or GSK3 inhibitors). Replicate samples were collected from each culture condition at 0, 24, 48 and 72 hours after differentiation commenced and analyzed for relative gene expression using real-time quantitative PCR. In addition, on day 3 of differentiation the cells were dissociated, labeled with phycoerythrin conjugated mouse anti-CXCR4 and the proportion of the cell population that was CXCR4-positive was determined using flow cytometry.

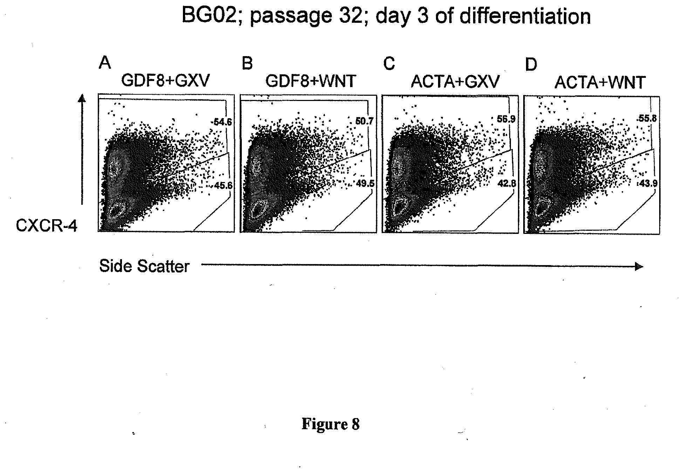

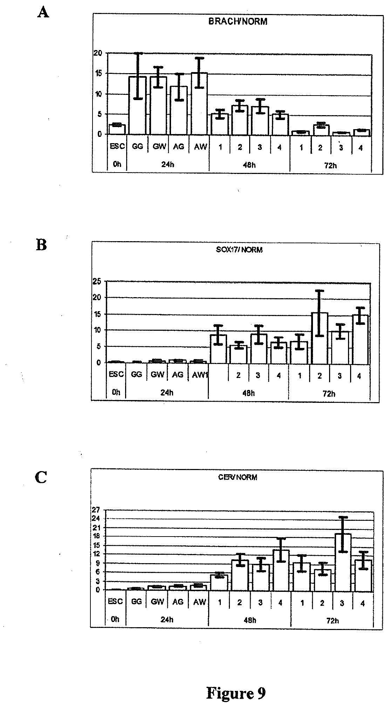

As shown in FIGs.8A-D, equivalent proportions of CXCR4-expressing definitive endoderm cells were produced by day 3 of differentiation with each of the 4 growth factor combinations used. For example, GDF8 combined with GSK3 inhibitor No.15 (GXV) produced about 54.6% CXCR4-positive definitive endoderm (FIG.8A); GDF8 combined with Wnt3a produced about 50.7% CXCR4-positive definitive endoderm (FIG.8B); Activin A combined with GSK3 inhibitor No.15 (GXV) produced about 56.9% CXCR4-positive definitive endoderm (FIG.8C); and Activin A combined with Wnt3a produced about 55.9% CXCR4-positive definitive endoderm (FIG.8D). Moreover, the hES cells transitioned through a brachyury-expressing mesendoderm intermediate en route to becoming SOX17 and CER1 expressing definitive endoderm expressing as shown by FIG.9.

The methods, compositions, and devices described herein are presently representative of preferred embodiments and are exemplary and are not intended as limitations on the scope of the invention.

As used in the claims below and throughout this disclosure, by the phrase "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

## Claims

1. A method for generating pancreatic endoderm cells, said method comprising:
(a) providing an *in vitro* cell population comprising pluripotent human cells with a factor selected from the group consisting of GDF8, GDF11 and a combination of GDF8 and GDF11 in an amount sufficient to induce pluripotent human cells of said cell population to differentiate into definitive endoderm cells, wherein said amount is at least 100 ng/ml GDF8 or at least 100 ng/ml GDF11 or at least 100 ng/ml of a combination of GDF8 and GDF11, and is sufficient to produce an *in vitro* cell population comprising at least 15% human definitive endoderm cells,
(b) further culturing the pluripotent-derived cells of step (a) to induce differentiation of the cells to pancreatic endoderm cells.

2. The method of claim 1, wherein at least 25%, at least 35%, at least 50%, at least 60%, at least 75%, at least 85%, or at least 95% of said pluripotent cells differentiate into definitive endoderm cells.

3. The method of claim 1 or 2, wherein said factor is provided in an amount sufficient to produce *in vitro* cell population comprising at least 25%, at least 35%, at least 50%, at least 60%, at least 75%, at least 85%, or at least 95% human definitive endoderm cells.

4. The method according to claim 1, further comprising providing to step (a) an agent which activates the Wnt3a signaling pathway, wherein the agent which activates the Wnt3a signaling pathway is selected from the group consisting of Wnt3a, a GSK3 inhibitor, a combination of GSK3 inhibitors and a combination of Wnt3a and one or more GSK3 inhibitors.

5. The method according to claim 4, said GSK3 inhibitor is selected from the group consisting of a pyridocarbazolo-cyclopentadienyl ruthenium complex, a dihydropyrimidine, and GSK3 inhibitor number 15.

6. The method of claim 1, wherein the pluripotent human cells are not contacted with an activin.

7. The method of claim 1, wherein the pluripotent cells are non-recombinant cells.

8. The method of any one of claims 1 to 7, wherein the pancreatic endoderm expresses a marker selected from the group comprising PDX1, PTF1A and NKX2.2.

9. An *in vitro* human cell culture comprising:
an *in vitro* cell population comprising human pluripotent cells and a factor selected from the group consisting of GDF8, GDF11 and a combination of GDF8 and GDF11; further comprising human definitive endoderm cells wherein at least 10% of the human cells in said cell culture are human definitive endoderm cells and wherein said factor is at least 100 ng/ml GDF8 or at least 100 ng/ml GDF11 or at least 100 ng/ml of a combination of GDF8 and GDF11; and pancreatic endoderm cells.

10. The cell culture of claim 9, wherein said pancreatic endoderm cells can differentiate into pancreatic islet hormone-expressing cells that express at least one pancreatic hormone.

11. The cell culture of claim 9, wherein said pancreatic endoderm cells express PDX1 and/or PTF1A.

12. The cell culture of claim 9, further comprising an agent which activates the Wnt3a signaling pathway to the first cell population, wherein the agent which activates the Wnt3a signaling pathway is selected from the group consisting of Wnt3a, a GSK3 inhibitor, a combination of GSK3 inhibitors and a combination of Wnt3a and one or more GSK3 inhibitors.

## Patentansprüche

1. Verfahren zur Generierung von Endodermzellen der Bauchspeicheldrüse, wobei das Verfahren umfasst:
(a) Versehen einer *in vitro*-Zellpopulation, die pluripotente humane Zellen umfasst, mit einem Faktor ausgewählt aus der Gruppe bestehend aus GDF8, GDF11 und einer Kombination aus GDF8 und GDF11 in einer Menge, die ausreichend ist, um pluripotente humane Zellen der Zellpopulation zu induzieren, sich in definitive Endodermzellen zu differenzieren, wobei die Menge mindestens 100ng/ml GDF8 oder mindestens 100ng/ml GDF11 oder mindestens 100 ng/ml einer Kombination aus GDF8 und GDF11 ist und ausreichend ist, um eine *in vitro*-Zellpopulation herzustellen, die mindestens 15% humane definitive Endodermzellen umfasst,
(b) Weiterzüchten der pluripotent abgeleiteten Zellen aus Schritt (a), um Differenzierung der Zellen in Endodermzellen der Bauchspeicheldrüse zu induzieren.

2. Verfahren nach Anspruch 1, wobei sich mindestens 25%, mindestens 35%, mindestens 50%, mindestens 60%, mindestens 75%, mindestens 85% oder mindestens 95% der pluripotenten Zellen in definitive Endodermzellen differenzieren.

3. Verfahren nach Anspruch 1 oder 2, wobei der Faktor in einer Menge bereitgestellt wird, die ausreichend ist, um eine *in vitro*-Zellpopulation herzustellen, die mindestens 25%, mindestens 35%, mindestens 50%, mindestens 60%, mindestens 75%, mindestens 85% oder mindestens 95% humane definitive Endodermzellen umfasst.

4. Verfahren nach Anspruch 1, des Weiteren umfassend das Bereitstellen eines Agens zu Schritt (a), das den Wnt3a-Signalweg aktiviert, wobei das Agens, das den Wnt3a-Signalweg aktiviert, ausgewählt ist aus der Gruppe bestehend aus Wnt3a, einem GSK3-Inhibitor, einer Kombination aus GSK3-Inhibitoren und einer Kombination aus Wnt3a und einem oder mehreren GSK3-Inhibitoren.

5. Verfahren nach Anspruch 4, wobei der GSK3-Inhibitor ausgewählt ist aus der Gruppe bestehend aus einem Pyridocarbazol-Cyclopentadienyl-Ruthenium-Komplex, einem Dihydropyrimidin und GSK3-Inhibitor Nummer 15.

6. Verfahren nach Anspruch 1, wobei die pluripotenten humanen Zellen nicht mit einem Activin in Kontakt gebracht werden.

7. Verfahren nach Anspruch 1, wobei die pluripotenten Zellen nicht-rekombinante Zellen sind.

8. Verfahren nach einem der Ansprüche 1 bis 7, wobei das Endoderm der Bauchspeicheldrüse einen Marker exprimiert, der aus der Gruppe ausgewählt ist, die PDX1, PTF1A und NKX2.2 umfasst.

9. *In vitro*-Kultur humaner Zellen, umfassend:
eine in *vitro*-Zellpopulation, umfassend humane pluripotente Zellen und einen Faktor, der ausgewählt ist aus der Gruppe bestehend aus GDF8, GDF11 und einer Kombination aus GDF8 und GDF11; des Weiteren umfassend humane definitive Endodermzellen, wobei mindestens 10% der humanen Zellen in der Zellkultur humane definitive Endodermzellen sind und wobei der Faktor mindestens 100ng/ml GDF8 oder mindestens 100 ng/ml GDF11 oder mindestens 100 ng/ml einer Kombination aus GDF8 und GDF11 ist; und Endodermzellen der Bauchspeicheldrüse.

10. Zellkultur nach Anspruch 9, wobei die Endodermzellen der Bauchspeicheldrüse sich in Langerhans-Insel-Hormon exprimierende Zellen (pancreatic islet hormon-expressing cells) differenzieren können, die mindestens ein Bauchspeicheldrüsenhormon exprimieren.

11. Zellkultur nach Anspruch 9, wobei die Endodermzellen der Bauchspeicheldrüse PDX1 und/oder PTF1A exprimieren.

12. Zellkultur nach Anspruch 9, des Weiteren umfassend ein Agens, das den Wnt3a-Signalweg zur ersten Zellpopulation aktiviert, wobei das Agens, das den Wnt3a-Signalweg aktiviert, ausgewählt ist aus der Gruppe bestehend aus Wnt3a, einem GSK3-Inhibitor, einer Kombination aus GSK3-Inhibitoren und einer Kombination aus Wnt3a und einem oder mehreren GSK3-Inhibitoren.

## Revendications

1. Méthode pour générer des cellules endodermiques pancréatiques, ladite méthode comprenant :
(a) la fourniture, à une population de cellules *in vitro* comprenant des cellules humaines pluripotentes, d'un facteur choisi dans le groupe constitué par GDF8, GDF11 et une combinaison de GDF8 et de GDF11 en une quantité suffisante pour induire des cellules humaines pluripotentes de ladite population de cellules afin qu'elles se différencient en cellules endodermiques définitives, ladite quantité étant d'au moins 100 ng/ml de GDF8 ou d'au moins 100 ng/ml de GDF11 ou d'au moins 100 ng/ml d'une combinaison de GDF8 et de GDF11, et étant suffisante pour produire une population de cellules *in vitro* comprenant au moins 15 % de cellules endodermiques définitives humaines;
(b) la culture supplémentaire des cellules dérivées de pluripotentes de l'étape (a) pour induire une différenciation des cellules en cellules endodermiques pancréatiques.

2. Méthode selon la revendication 1, dans laquelle au moins 25 %, au moins 35 %, au moins 50 %, au moins 60 %, au moins 75 %, au moins 85 %, ou au moins 95 % desdites cellules pluripotentes se différencient en cellules endodermiques définitives.

3. Méthode selon la revendication 1 ou 2, dans laquelle ledit facteur est fourni en une quantité suffisante pour produire une population cellulaire *in vitro* comprenant au moins 25 %, au moins 35 %, au moins 50 %, au moins 60 %, au moins 75 %, au moins 85 % ou au moins 95 % de cellules endodermiques définitives humaines.

4. Méthode selon la revendication 1, comprenant en outre la fourniture à l'étape (a) d'un agent qui active la voie de signalisation de Wnt3a, ledit agent qui active la voie de signalisation de Wnt3a étant choisi dans le groupe constitué par Wnt3a, un inhibiteur de GSK3, une combinaison d'inhibiteurs de GSK3 et une combinaison de Wnt3a et d'un ou plusieurs inhibiteurs de GSK3.

5. Méthode selon la revendication 4, dans laquelle ledit inhibiteur de GSK3 est choisi dans le groupe constitué par un complexe de pyridocarbazolo-cyclopentadiényl-ruthénium, une dihydropyrimidine, et l'inhibiteur de GKS3 numéro 15.

6. Méthode selon la revendication 1, dans laquelle les cellules humaines pluripotentes ne sont pas mises en contact avec une activine.

7. Méthode selon la revendication 1, dans laquelle les cellules pluripotentes sont des cellules non recombinées.

8. Méthode selon l'une quelconque des revendications 1 à 7, dans laquelle l'endoderme pancréatique exprime un marqueur choisi dans le groupe constitué par PDX1, PTF1A et NKX2.2.

9. Culture de cellules humaines *in vitro* comprenant :
une population de cellules *in vitro* comprenant des cellules humaines pluripotentes et un facteur choisi dans le groupe constitué par GDF8, GDF11 et une combinaison de GDF8 et de GDF11 ; comprenant en outre des cellules endodermiques définitives humaines, dans laquelle au moins 10 % des cellules humaines dans ladite culture de cellules sont des cellules endodermiques définitives humaines, et dans laquelle ledit facteur est d'au moins 100 ng/ml de GDF8 ou d'au moins 100 ng/ml de GDF11 ou d'au moins 100 ng/ml d'une combinaison de GDF8 et de GDF11 ; et des cellules endodermiques pancréatiques.

10. Culture de cellules selon la revendication 9, dans laquelle les cellules endodermiques pancréatiques peuvent se différencier en cellules exprimant des hormones d'îlots pancréatiques qui expriment au moins une hormone pancréatique.

11. Culture de cellules selon la revendication 9, dans laquelle lesdites cellules endodermiques pancréatiques expriment PDX1 et/ou PTF1A.

12. Culture de cellules selon la revendication 9, comprenant en outre un agent qui active la voie de signalisation de Wnt3a à la première population de cellules, dans laquelle l'agent qui active la voie de signalisation de Wnt3a est choisi dans le groupe constitué par Wnt3a, un inhibiteur de GSK3, une combinaison d'inhibiteurs de GSK3 et une combinaison de Wnt3a et d'un ou plusieurs inhibiteurs de GSK3.
